(19)

**Europäisches Patentamt**
**European Patent Office**
**Office européen des brevets**

(11) **EP 4 678 170 A1**

(12) **EUROPEAN PATENT APPLICATION**

(43) Date of publication:
**14.01.2026 Bulletin 2026/03**

(21) Application number: **24189576.2**

(22) Date of filing: **18.07.2024**

(51) International Patent Classification (IPC):
*A61K 31/137* (2006.01)   *A61K 9/00* (2006.01)
*A61K 31/56* (2006.01)   *A61K 31/58* (2006.01)
*A61M 15/00* (2006.01)   *A61P 11/06* (2006.01)

(52) Cooperative Patent Classification (CPC):
(C-Sets available)
**A61K 31/137; A61K 9/0075; A61K 31/56;**
**A61K 31/58; A61M 15/0065; A61P 11/06;**
A61M 2202/064                                (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **08.07.2024 US 202463668628 P**

(71) Applicant: **Norton (Waterford) Limited**
**Waterford, X91 WK68 (IE)**

(72) Inventor: **SMALL, Calvin J**
**Philadelphia (US)**

(74) Representative: **Elkington and Fife LLP**
**Prospect House**
**8 Pembroke Road**
**Sevenoaks, Kent TN13 1XR (GB)**

(54) **A FIXED-DOSE DRY POWDER INHALATION FORMULATION OF FLUTICASONE PROPIONATE AND ALBUTEROL SULFATE FOR THE TREATMENT OF ASTHMA**

(57)    This invention relates to a method of treatment of asthma comprising a pro re nata (PRN) administration of a fixed-dose dry powder inhalation composition comprising fluticasone propionate and albuterol sulfate as a rescue medication in patients, wherein the fluticasone propionate is administered at a delivered dose of 22-59 mcg per inhalation and the albuterol sulfate is administered at a delivered dose of 92-124 mcg per inhalation, and wherein the treatment provides an improved baseline-adjusted postdose forced expiratory volume in one second ($FEV_1$) area under the effect curve from time zero to 6 hours ($AUEC_{0-6h}$) when compared to fluticasone propionate treatment and an improved baseline-adjusted trough $FEV_1$ when compared to albuterol sulfate treatment.

**EP 4 678 170 A1**

(52) Cooperative Patent Classification (CPC): (Cont.)

C-Sets
**A61K 31/137, A61K 2300/00;
A61K 31/58, A61K 2300/00**

**Description**

**Background of the invention**

This invention relates to an inhalable formulation, and particularly to a fixed-dose composition of fluticasone propionate and albuterol sulfate for the treatment of asthma.

Asthma is one of the most common chronic diseases. It is a heterogeneous condition that affects the airways of the lungs and is characterized by airway inflammation and bronchial hyper-responsiveness, resulting in variable reductions in expiratory airflow that vary over time in their occurrence, frequency, and intensity. During acute asthmatic episodes, the airway passages become narrower and more obstructed, resulting in coughing, wheezing, tightness of the chest, shortness of breath, and increased mucus production. In some subjects, asthma may lead to chronic irreversible changes in airway structure and function, increasing morbidity and mortality. This progressive decline in lung function can be observed by measuring the forced expiratory volume in one second ($FEV_1$) over time.

A decline in lung function increases death rates, limits a patient's ability to engage in routine activities of daily living and reduces quality of life.

Inhaled corticosteroids and $\beta_2$-agonists represent two classes of active ingredient that have been developed to treat respiratory disorders, particularly asthma. Each class has differing targets and effects.

Inhaled corticosteroids (ICSs) are steroid hormones used in the long-term control of respiratory disorders. They function by reducing the airway inflammation, controlling symptoms and reducing future risks of exacerbation and lung function decline. They are often termed "controller" or "maintenance" medicines.

Presently marketed ICSs are for example beclomethasone dipropionate, budesonide, ciclesonide, fluticasone furoate, fluticasone propionate and mometasone furoate. The compounds are all well-known in the art. For example, fluticasone propionate is named as S-(fluoromethyl)-6$\alpha$,9-difluoro-11$\beta$,17-dihydroxy-16$\alpha$-methyl-3-oxoandrosta-1,4-diene-17$\beta$-carbothioate-17-propanoate.

Short-acting $\beta_2$-agonists (SABAs) are examples of bronchodilators, and are employed to dilate the bronchi and bronchioles, decreasing resistance in the airways, and thereby increasing the airflow to the lungs. Bronchodilators may be short-acting or long-acting. Short-acting bronchodilators provide a rapid but short-term relief from acute bronchoconstriction (and are often called "rescue" or "reliever" medicines), whereas long-acting bronchodilators typically help control symptoms over a longer period of time compared to SABAs.

SABAs are used acutely to provide rapid symptom relief and can be lifesaving, but they do not address underlying airway inflammation. When patients experience worsening asthma symptoms, they gain immediate relief with SABA medication and therefore increase its use. However, patients generally do not increase the use of ICS in a similar manner. SABA overuse, defined as use of ≥3 canisters per year is common and is a risk factor for poor asthma outcomes and severe exacerbation.

Albuterol (also known as salbutamol) is a short-acting $\beta_2$-agonist that is indicated for the treatment or prevention of bronchospasm in patients with asthma. It is named as 4-[2-(tert-butylamino)-1-hydroxyethyl]-2-(hydroxymethyl)-phenol. Albuterol is the usual bronchodilator used in acute asthma management. It is typically administered as the sulfate salt, the structure of which is well-known in the art.

According to the Global Initiative for Asthma (GINA) 2023 guidelines, a step-wise approach is taken to treatment. For adults and adolescents (aged ≥12 years), the approach is divided into track 1 and track 2.

In track 1, at steps 1-2, which represent a mild form of asthma, the patient is given an as-needed low-dose ICS-formoterol (a long-acting $\beta_2$ adrenoceptor agonist (LABA) with a rapid onset of action). At step 3, a maintenance low-dose ICS-formoterol is given. At step 4, this is increased to a medium-dose ICS-formoterol. At step 5, an add on LAMA (long-acting muscarinic antagonist) is included, and a high-dose ICS-formoterol is considered. Over all steps, the reliever is an as-needed low-dose ICS-formoterol. In track 2, the reliever is an as-needed SABA or an as-needed ICS-SABA. Step 1 is to take an ICS whenever the SABA is taken. Step 2 adds a low-dose maintenance ICS. Step 3 is a low-dose maintenance ICS-LABA. Step 4 is a medium/high-dose ICS-LABA. At step 5, an add on LAMA is included, and a high-dose ICS-LABA is considered.

A similar approach for pediatric patients (aged 6-11) is recommended in the GINA guidelines. Step 1 is to take an ICS whenever the SABA is taken. Step 2 adds a low-dose maintenance ICS. Step 3 is a low-dose maintenance ICS-LABA, or medium-dose maintenance ICS, or very low dose ICS-formoterol maintenance and reliever (MART). Step 4 is a medium-dose ICS-LABA or low dose ICS-formoterol maintenance and reliever (MART). At step 5, a high-dose ICS-LABA or add-on therapy is considered. The reliever is an as-needed SABA or low-dose ICS-formoterol for MART in steps 3 and 4.

Interestingly, despite the availability of the present therapies, the majority of asthmatic patients remain poorly controlled. There are several likely contributing causes of poor control one of which is poor adherence to prescribed maintenance therapies. This is a contributor to excess morbidity and mortality of asthma.

Despite the benefits of regular daily maintenance treatment, poor adherence is commonplace and associated with significant asthma-related morbidity. Most patients with persistent asthma take for example ICS therapy infrequently, only using 2 to 4 canisters per year. Thus, there remains a need in the art for improving the treatment of asthma patients, particularly with reference to lung function, as improving lung function decreases death rates and improves quality of life.

## Summary of the invention

Accordingly, the present invention provides a method of treatment of asthma comprising a pro re nata (PRN) administration of a fixed-dose dry powder inhalation composition comprising fluticasone propionate and albuterol sulfate as a rescue medication in patients, wherein the fluticasone propionate is administered at a delivered dose of 22-59 mcg per inhalation and the albuterol sulfate is administered at a delivered dose of 92-124 mcg per inhalation, and wherein the treatment provides an improved baseline-adjusted postdose forced expiratory volume in one second ($FEV_1$) area under the effect curve from time zero to 6 hours ($AUEC_{0-6h}$) when compared to fluticasone propionate treatment and an improved baseline-adjusted trough $FEV_1$ when compared to albuterol sulfate treatment.

The present invention also provides a fixed-dose dry powder inhalation composition comprising fluticasone propionate and albuterol sulfate for use in the treatment of asthma comprising a pro re nata (PRN) administration of the fixed-dose dry powder inhalation composition comprising fluticasone propionate and albuterol sulfate as a rescue medication in patients, wherein the fluticasone propionate is administered at a delivered dose of 22-59 mcg per inhalation and the albuterol sulfate is administered at a delivered dose of 92-124 mcg per inhalation, and wherein the treatment provides an improved baseline-adjusted postdose forced expiratory volume in one second ($FEV_1$) area under the effect curve from time zero to 6 hours ($AUEC_{0-6h}$) when compared to fluticasone propionate treatment and an improved baseline-adjusted trough $FEV_1$ when compared to albuterol sulfate treatment.

In a preferred embodiment, the fixed-dose dry powder inhalation composition consists of fluticasone propionate, albuterol sulfate and lactose. Preferably, the lactose is alpha-lactose monohydrate.

The method of treatment of the present invention provides a significant improvement in the asthma treatment of patients, and in particular to the decrease in the reduction in lung function.

## Brief description of the drawings

The invention will now be described in detail with reference to the accompanying drawings, in which:

Fig. 1 is a front perspective view of a dry powder inhaler according to a preferred embodiment;
Fig. 2 is a cross-sectional interior perspective view of the inhaler shown in Fig. 1;
Fig. 3 is an exploded perspective view of the example inhaler shown in Fig. 1;
Fig. 4 is an exploded perspective view of a top cap and electronics module of the inhaler shown in Fig.1;
Fig. 5 is an exploded isometric view of a deagglomerator according to the present disclosure;
Fig. 6 is a side elevation view of the deagglomerator of Fig. 5;
Fig. 7 is a top plan view of the deagglomerator of Fig. 5;
Fig. 8 is a bottom plan view of the deagglomerator of Fig. 5;
Fig. 9 is a sectional view of the deagglomerator of Fig. 5 taken along line 5'-5' of Fig. 6; and
Fig. 10 is a sectional view of the deagglomerator of Fig. 5 taken along line 6'-6' of Fig. 7.

## Detailed description

This invention relates to a method of treating asthma by administering a fixed-dose dry powder inhalation composition containing the APIs, fluticasone propionate and albuterol sulfate.

In its broadest embodiment, the indication being treated is asthma. The asthma treatment includes treating or preventing bronchospasm in patients with reversible obstructive airways disease and/ or preventing exacerbations.

In a preferred embodiment, the fixed-dose dry powder inhalation composition consists of fluticasone propionate, albuterol sulfate and lactose. Preferably, the lactose is alpha-lactose monohydrate.

The individual APIs are also known in the art and have already been discussed in the context of the background the present invention hereinabove.

The present invention relates to a fixed-dose combination (FDC), meaning that both APIs are present in the same formulation, e.g. a dry powder formulation. When referring to dry powder medicaments, both medicaments can be contained in a reservoir of the inhaler, in capsules or in blisters. Preferably, the two APIs are formulated together providing a fixed dose dry powder composition contained in the inhaler reservoir.

In capsules or blister strips, the two APIs can be in the same or separate/different capsule/blister pocket as long as they are contained in the same device. Such inhalers are well known in the art, and are also described in more detail hereinbelow. It should be noted that when the medicament is contained in previously measured amounts in individual containers, e.g. a capsule or a blister, the metered dose may be termed a "pre-metered" dose.

The fixed dose combination of the present invention is administered pro re nata (PRN) as a rescue medicine, which means it is not used as maintenance therapy. Indeed, the patients can separately administer a maintenance dose of any kind of long-term asthma medication such as ICS, LABA, LAMA (long-acting muscarinic antagonist), SAMA (short-acting muscarinic antagonist), various biological medications and combinations thereof. The ICS can be the same as in the fixed-dose dry powder inhalation composition, i.e. fluticasone propionate, it can be another ICS, a combination of ICSs, or a combination of ICS with any of the above listed actives. Indeed, an advantage of the present invention is that the present combination may be used as rescue medication with any background asthma maintenance treatment or with no background treatment.

Examples of maintenance therapies which may be used with the present invention include ICSs, such as flunisolide, fluticasone propionate (approved products are the HFA pMDI and DPI e.g. ArmonAir®/AirDuo®), fluticasone furoate (approved product is the DPI), beclomethasone dipropionate (approved products are the DPI e.g. Easyhaler® and the HFA pMDI, e.g. Qvar®), budesonide (approved product is the DPI), mometasone furoate (approved products are the DPI and HFA pMDI), ciclesonide (approved product is the HFA pMDI) and combinations thereof.

Examples of LABAs include formoterol, arformoterol, salmeterol, bambuterol, clenbuterol and combinations thereof. Ultra-LABAs may also be used, e.g. abediterol, indacaterol, olodaterol, vilanterol, carmoterol and combinations thereof.

Examples of LAMAs include tiotropium, glycopyrronium, umeclidinium, aclidinium, darotropium and combinations thereof. As SAMA, an example is ipratropium.

Examples of biological maintenance therapies that may be used with the present invention include tezepelumab, dupilumab, mepolizumab, reslizumab, benralizumab, omalizumab, palivizumab and combinations thereof.

The maintenance therapy daily metered dose can be low-, medium- or high-dose depending on the type of medicament and patient's age. For example, for patients aged 12 years and older, the daily metered doses are as follows. Beclomethasone dipropionate, low-dose is 200-500 mcg, medium-dose is >500-1,000 mcg, high-dose is >1,000 mcg. Beclomethasone dipropionate ultra-fine particle, low-dose is 100-200 mcg, medium-dose is >200-400 mcg, high-dose is >400 mcg. Budesonide, low-dose is 200-400 mcg, medium-dose is >400-800 mcg, high-dose is >800 mcg. Ciclesonide, low-dose is 80-160 mcg, medium-dose is >160-320 mcg, high-dose is >320 mcg. Fluticasone furoate, low-dose and medium-dose are 100 mcg, high-dose is 200 mcg. Fluticasone propionate, low-dose is 100-250 mcg, medium-dose is >250-500 mcg, high-dose is >500 mcg. Mometasone furoate, low-dose and medium-dose are 200-400 mcg, high-dose is >400 mcg.

For patients aged 6 to <12 years old the ICS daily metered doses are as follows. Beclomethasone dipropionate, low-dose is 100-200 mcg, medium-dose is >200-400 mcg, high-dose is >400 mcg. Beclomethasone dipropionate ultra-fine particle, low-dose is 50-100 mcg, medium-dose is >100-200 mcg, high-dose is >200 mcg. Budesonide, low-dose is 100-200 mcg, medium-dose is >200-400 mcg, high-dose is >400 mcg. Budesonide nebules, low-dose is 250-500 mcg, medium-dose is >500-1,000 mcg, high-dose is >1,000 mcg. Ciclesonide, low-dose is 80 mcg, medium-dose is >80-160 mcg, high-dose is >160 mcg. Fluticasone furoate, low-dose and medium-dose are 50 mcg. Fluticasone propionate, low-dose is 50-100 mcg, medium-dose is >100-200 mcg, high-dose is >200 mcg. Mometasone furoate, low-dose and medium-dose are 100 mcg, high-dose is 200 mcg.

Other maintenance therapies can be found in the GINA guidelines and are provided at the approved or recommended doses. For example, in track 1, steps 1-2 budesonide-formoterol is given at a metered dose of 200/6 mcg (delivered 160/4.5 mcg) for patients 12 years and above. Track 1, steps 3-4 is aged 6-11 years budesonide-formoterol metered dose of 100/6 mcg (delivered 80/4.5 mcg), aged 12-17 years budesonide-formoterol metered dose of 200/6 mcg (delivered 160/4.5 mcg), aged 18 years and over budesonide-formoterol metered dose of 200/6 mcg (delivered 160/4.5 mcg) or beclomethasone-formoterol metered dose of 100/6 mcg (delivered 84.6/5.0 mcg). Track 1, step 5 is aged 12-17 years budesonide-formoterol metered dose of 200/6 mcg (delivered 160/4.5 mcg), aged 18 years and over budesonide-formoterol metered dose of 200/6 mcg (delivered 160/4.5 mcg) or beclomethasone-formoterol metered dose of 100/6 mcg (delivered 84.6/5.0 mcg).

The method of treatment of asthma of the present invention comprises PRN administration as rescue medication of a fixed dose combination of fluticasone propionate and albuterol sulfate. The fluticasone propionate is administered at a delivered dose of 22-59 mcg per inhalation and the albuterol sulfate is administered at a delivered dose of 92-124 mcg per inhalation. Preferably, the fluticasone propionate is administered at a delivered dose of 23-56 mcg per inhalation and the albuterol sulfate is administered at a delivered dose of 97-119 mcg per inhalation. More preferably, the fluticasone propionate is administered at a delivered dose of 25-54 mcg per inhalation and the albuterol sulfate is administered at a delivered dose of 103-113 mcg per inhalation. In a particular embodiment, the fluticasone propionate is administered at a delivered dose of 26 or 51 mcg per inhalation and the albuterol sulfate is administered at a delivered dose of 108 mcg per inhalation.

In addition, the fluticasone propionate is typically administered at a metered dose of 26-63 mcg per inhalation and the albuterol sulfate is administered at a metered dose of 99-135 mcg per inhalation. Preferably, the fluticasone propionate is administered at a metered dose of 27-61 mcg per inhalation and the albuterol sulfate is administered at a metered dose of 105-129 mcg per inhalation. More preferably, the fluticasone propionate is administered at a metered dose of 29-58 mcg per inhalation and the albuterol sulfate is administered at a metered dose of 111-123 mcg per inhalation. In a particular embodiment, the fluticasone propionate is administered at a metered dose of 30 or 55 mcg per inhalation and the albuterol sulfate is administered at a metered dose of 117 mcg per inhalation.

Typically, the PRN maximum daily metered dose does not exceed 6 doses, i.e. 12 inhalations (2 inhalations per dose).

The terms "metered dose" and "delivered dose" are conventional terms in the art, and have their standard meanings. That is, the metered dose is the mass of active that is available within the aerosol generator per actuation. This is the dose that is metered. The delivered dose is the mass of the active emitted per actuation that is actually available for inhalation at the mouth.

The method of treating asthma of the present invention in which the fixed-dose combination of fluticasone propionate and albuterol sulfate is used as reliever treatment improves lung function for patients with asthma (improving lung function in asthma patients typically means decreasing the reduction in lung function). While many patients are treated with ICS or ICS/LABA combinations as part of their maintenance therapy, many are noncompliant with this treatment. Multiple studies have shown that many of the asthma patients are noncompliant with their maintenance therapy. As patients' asthma deteriorates, they become symptomatic which prompts the use of more rescue medication. With the incorporation of ICS, in particular fluticasone propionate, and albuterol sulfate into a single inhaler, the patients gain relief of symptoms, but the combination will also treat the underlying inflammation that is responsible for the increase in symptoms and thus improve lung function compared to albuterol alone rescue treatment (PRN administration). Thus, the incorporation of ICS, in particular fluticasone propionate, and albuterol sulfate into a single inhaler minimizes the untoward effect of overuse of albuterol (or the single enantiomer, levalbuterol) administered as the sole rescue therapy (e.g. exacerbation, hospitalization and death).

Reducing the exposure to corticosteroid is also an important advantage to this approach. The short-term increase in ICS exposure is self-limited since the patient will either improve and use less rescue therapy, and thus less ICS, or the patient will present for treatment of a severe exacerbation, resulting in systemic corticosteroid treatment and higher exposure. In a sense, the patient self-titrates based on the requirement to treat asthma symptoms.

A fixed-dose dry powder inhalation composition of the present invention is used in the treatment of asthma. The treatment includes prevention of bronchospasm in patients with reversible obstructive airway disease and /or prevention of exacerbations, wherein the fixed dose dry powder inhalation composition is PRN administered as a rescue medication and wherein it comprises fluticasone propionate and albuterol sulfate. Thus, the present invention provides a method for treating asthma, the method includes treating or preventing bronchospasm in patients with reversible obstructive airways disease and/or preventing exacerbations comprising pro re nata (PRN) administration of a fixed-dose dry powder inhalation composition comprising fluticasone propionate and albuterol sulfate as a rescue medication.

The postdose forced expiratory volume in one second ($FEV_1$) area under the effect curve from time zero to 6 hours ($AUEC_{0-6h}$) is to assess the contribution of the albuterol sulfate in decreasing the reduction in lung function and improving (i.e. decreasing) reductions in airflow typically seen in poorly controlled asthma, and hence is compared to fluticasone propionate treatment alone (i.e. as a monotherapy), typically the same dose of fluticasone propionate given four times per day (2 inhalation 4 times daily). The baseline-adjusted trough $FEV_1$ is to assess the contribution of the fluticasone propionate in decreasing the reduction in lung function and improving (i.e. decreasing) reductions in airflow typically seen in poorly controlled asthma, and hence is compared to the albuterol sulfate rescue therapy alone (i.e. as a monotherapy), typically the same dose of albuterol sulfate also given prn, i.e. 2 inhalations four times daily. In both cases, an improvement over placebo is also observed, wherein placebo is also administered 2 inhalations 4 times daily. Specifically, the method/composition of the present invention provides an improved baseline-adjusted postdose forced expiratory volume in one second ($FEV_1$) area under the effect curve from time zero to 6 hours ($AUEC_{0-6h}$) when compared to fluticasone propionate treatment and an improved baseline-adjusted trough $FEV_1$ when compared to albuterol sulfate treatment. In a preferred embodiment the treatment means administering either fluticasone propionate or albuterol sulfate 2 inhalations 4 times daily.

In a specific embodiment, the asthma treatment provides a decrease in the reduction of the lung function as reflected by a baseline-adjusted postdose forced expiratory volume in one second ($FEV_1$) area under the effect curve from time zero to 6 hours ($AUEC_{0-6h}$) of at least 100 mL, preferably at least 150 mL compared to placebo. The maximum value is less relevant, but may be 300 mL. Typically, this effect is observed within 12 weeks of commencing the treatment, and preferably already within 6 weeks or even within 4 weeks of commencing the treatment.

In a specific embodiment, the asthma treatment provides a decrease in the reduction of the lung function as reflected by an increase in baseline-adjusted trough $FEV_1$ of at least 100 mL compared to placebo, preferably at least 150 mL and most preferably at least 200 mL. Again, the maximum value is less relevant, but may be 400 mL. Typically, this effect is observed within 12 weeks of commencing the treatment, and preferably already within 6 weeks or even within 4 weeks of commencing the treatment.

In a preferred embodiment, the decrease in the reduction of the lung function as reflected by the baseline-

adjusted postdose $FEV_1$ $AUEC_{0-6h}$ and the increase in baseline-adjusted trough $FEV_1$ are both at least 100 mL, compared to placebo. Alternatively, these values may be at least 150 mL together with at least 200 mL, respectively. Again, this effect is typically observed within 12 weeks of commencing the treatment, and preferably already within 6 weeks or even within 4 weeks of commencing the treatment.

The method/composition of the present invention is for prn use. However, the efficacy of the method/composition of the present invention is assessed in the clinical trial described hereinbelow using administration four times per day, which corresponds to the approximate duration of the bronchodilatory effect for albuterol (albuterol sulfate). This is intended to mimic prn administration, but gives the necessary standardization by which the results of the trial may be assessed. Thus, in an embodiment of the present invention, the efficacy of the method/composition is determined by administering the fixed-dose dry powder inhalation composition comprising fluticasone propionate and albuterol sulfate four times per day. The comparison is with fluticasone propionate as a monotherapy given four times per day and albuterol sulfate as a monotherapy given four times per day. In a preferred embodiment the fluticasone propionate monotherapy is a dry powder inhaler for example Armonair Respiclik, and the albuterol sulfate comparison therapy is a dry powder inhaler for example ProAir Respiclick.

The fluticasone propionate known/available therapy is typically a maintenance therapy and the albuterol sulfate known/available therapy is typically a rescue therapy. The PRN administration of the fixed-dose combination of the present invention provides an improvement in the lung function parameters defined herein compared to the fluticasone propionate maintenance therapy combined with a separate albuterol sulfate rescue therapy.

Albuterol sulfate rescue treatment as used herein refers to a PRN administration of albuterol sulfate only, wherein the patient can be also on a separate maintenance treatment of any kind of long-term asthma medication such as ICS, LABA, LAMA, SAMA, various biological medications, and combinations thereof. The preferred metered dose of albuterol sulfate is 117 mcg (and a 108 mcg delivered dose). If an ICS is used as maintenance treatment it may be administered as a monoproduct, or in combination with other long-term asthma drugs. The ICS can be the same as in the fixed-dose dry powder inhalation composition, i.e. fluticasone propionate, it can be another ICS, or a combination of ICS with any of the above listed actives as described hereinabove.

Examples of commercially available fluticasone propionate products are: Flovent® HFA 88, 220, 440 and 880 twice a day delivered dose; Flixotide® (EU) 100, 250, 500 twice a day metered dose; Flovent® Discus® 50, 100, 250 twice a day metered dose; and Armonair® 30, 55, 113 and 232 twice a day metered dose.

In another embodiment, the method/composition of the present invention provides one or more of: a reduced time to 15% improvement from baseline $FEV_1$ postdose when compared to albuterol sulfate treatment; a reduced time to 12% improvement from baseline $FEV_1$ postdose when compared to albuterol sulfate treatment; an increased duration of 15% increase in $FEV_1$ from baseline postdose when compared to albuterol sulfate treatment; an improved Asthma Control Questionnaire-6 (ACQ-6) response defined as achieving a decrease in score from baseline value of at least 0.5 when compared to albuterol sulfate treatment; and/or an improved Asthma Control Test (ACT) score response defined as achieving an increase in score from baseline value of at least 3 when compared to albuterol sulfate treatment. In a preferred embodiment the comparison albuterol sulfate treatment is 2 inhalations 4 times daily. In a more preferred embodiment, the comparison albuterol sulfate therapy is a dry powder inhaler for example ProAir Respiclick.

In another embodiment, the method/composition of the present invention provides one or more of: a reduced time to 15% improvement from baseline $FEV_1$ postdose when compared to fluticasone propionate treatment; a reduced time to 12% improvement from baseline $FEV_1$ postdose when compared to fluticasone propionate treatment; an increased duration of 15% increase in $FEV_1$ from baseline postdose when compared to fluticasone propionate treatment; an improved Asthma Control Questionnaire-6 (ACQ-6) response defined as achieving a decrease in score from baseline value of at least 0.5 when compared to fluticasone propionate treatment; and/or an improved Asthma Control Test (ACT) score response defined as achieving an increase in score from baseline value of at least 3 when compared to fluticasone propionate treatment. In a preferred embodiment the comparison fluticasone propionate treatment is 2 inhalations 4 times daily. In a more preferred embodiment, the comparison fluticasone propionate therapy is a dry powder inhaler for example Armonair Respiclick.

In another embodiment, the method/composition of the present invention provides one or more of: a reduced time to 15% improvement from baseline $FEV_1$ postdose when compared to placebo treatment; a reduced time to 12% improvement from baseline $FEV_1$ postdose when compared to placebo treatment; an increased duration of 15% increase in $FEV_1$ from baseline postdose when compared to placebo treatment; an improved Asthma Control Questionnaire-6 (ACQ-6) response defined as achieving a decrease in score from baseline value of at least 0.5 when compared to placebo treatment; and/or an improved Asthma Control Test (ACT) score response defined as achieving an increase in score from baseline value of at least 3 when compared to placebo treatment. In a preferred embodiment the comparison placebo treatment is 2 inhalations 4 times daily.

In another embodiment the method/composition of the present invention provides one or more of: a reduced time to 15% improvement from baseline $FEV_1$ postdose when compared to placebo treatment, fluticasone propionate treatment or albuterol sulfate treatment; a reduced time to 12% improvement from baseline $FEV_1$ postdose when

compared to placebo treatment, fluticasone propionate treatment or albuterol sulfate treatment; an increased duration of 15% increase in $FEV_1$ from baseline postdose when compared to placebo treatment, fluticasone propionate treatment or albuterol sulfate treatment; an improved Asthma Control Questionnaire-6 (ACQ-6) response defined as achieving a decrease in score from baseline value of at least 0.5 when compared to placebo treatment, fluticasone propionate treatment or albuterol sulfate treatment; and/or an improved Asthma Control Test (ACT) score response defined as achieving an increase in score from baseline value of at least 3 when compared to placebo treatment, fluticasone propionate treatment or albuterol sulfate treatment. In a preferred embodiment the comparison albuterol sulfate, fluticasone propionate and placebo treatments is 2 inhalations 4 times daily. In a more preferred embodiment, the comparison fluticasone propionate therapy is a dry powder inhaler for example Armonair Respiclick and the albuterol sulfate therapy is a dry powder inhaler for example ProAir Respiclick.

In an embodiment of the present invention, the treatment is in patients ≥4 years old. In a preferred embodiment the group of patients 4 years and older includes patients 4 to <18 years old and patients 18 years and older. Patients 4 to <18 years old include one of ≥4 to <12-year-old patients, ≥6 to <12-year-old patients, ≥12 to <18-year-old patients and combination thereof. In a more preferred embodiment, the ≥4 to <12-year-old patient age group includes at least one of 4-11 and 6-11 years old.

The present invention may be applied to patients at any of the GINA steps 1-5, i.e. the patients are any one of GINA step 1-5 patients.

The fixed-dose dry powder inhalation composition of the present invention can be provided for example in two doses. One dose product is administered with an exemplified metered dose of 30 mcg fluticasone propionate and 117 mcg albuterol sulfate per inhalation (equivalent to 26 mcg and 108 mcg delivered dose per inhalation, respectively). The second dose product is administered with an exemplified metered dose of 55 mcg fluticasone propionate and 117 mcg albuterol sulfate per inhalation (equivalent to 51 mcg and 108 mcg delivered dose per inhalation, respectively). The amounts of albuterol sulfate set out herein, e.g. 117 mcg metered and 108 mcg delivered, are based on the total weight of the salt. These specific values correspond to 97.5 mcg and 90 mcg, respectively, based on the albuterol free base. Bioequivalent doses to the doses of the present invention are also included, particularly doses bioequivalent to the delivered doses, as described herein.

The fixed-dose dry powder inhalation composition of the present invention is a dry powder formulation for inhalation. That means the product is provided with a powder carrier, such as lactose, particularly α-lactose monohydrate.

Such carriers are termed "coarse" carriers to distinguish them from fine particles which are entrained into the lung. They are well known in the art and are readily available commercially from a number of sources. A coarse carrier usually contains some fine particles of the same material (inherently present and/or deliberately added). Such fine particles assist with the release of the active ingredient(s) from the coarse carrier

In general, the particle size of the α-lactose monohydrate carrier should be such that it can be entrained in an air stream but not deposited in the key target sites of the lung. Accordingly, the α-lactose monohydrate preferably has the following particle size distribution as measured by laser diffraction: d10 is 20-40 μm; d50 is 55-65 μm; and d90 is 80-100 μm.

The lactose may contain inherent fine content (i.e. fine lactose) or they may also be deliberately added to the formulation. Such lactose has a particle size less than 10 μm in size, more likely 1-5 μm as measured by laser diffraction. In a preferred embodiment the % w/w of the particles that is smaller than 10 μm is less than 6 as measured by laser diffraction.

Dry powder inhalable formulations may also contain a ternary excipient. Ternary excipients are well-known in the art and are used for example to provide additional stability to the active ingredients. Typically, the additional stability is provided by reducing the amount of water adsorption and by promoting release of the active ingredient from the coarse carrier particles.

Ternary excipients are also known as force control agents, lubricants or anti-adherents. They use the term "ternary" because they add a third material to the formulation over the active ingredient(s) and the carrier. It should be noted that the coarse carrier (i.e. α-lactose monohydrate) usually contains some fine particles of the same material (inherently present and/or deliberately added). Such fine particles composed of the same material as the coarse carrier are not ternary excipients.

Ternary excipients are also known as force control agents, lubricants or anti-adherents. They use the term "ternary" because they add a third material to the formulation over the active ingredient(s) and the carrier. It should be noted that the coarse carrier (i.e. α-lactose monohydrate) usually contains some fine particles of the same material (inherently present and/or deliberately added). Such fine particles composed of the same material as the coarse carrier are not ternary excipients.

In one embodiment, the dry powder inhalable formulation of the present invention does not include a ternary excipient. For example, the formulation may consist of fluticasone propionate, albuterol sulfate and lactose (e.g. an α-lactose monohydrate carrier, optionally containing fine α-lactose monohydrate particles).

In another embodiment, the dry powder inhalable formulation of the present invention further comprises a ternary excipient.

Typical examples of ternary excipients which may be formulated within the formulation of the present invention include metal stearates (such as magnesium and calcium stearate), fatty acids (e.g. stearic acid), amino acids (such as leucine) and phospholipids (such as lecithin).

It is preferred wherein the ternary excipient formulated within the formulation of the present invention is magnesium stearate. It is also preferred wherein the proportion of magnesium stearate contained within the formulation is 0.01-3.0% w/w by weight of the formulation. Ternary excipients can be used to provide additional stability.

The fixed dose dry powder composition of the present invention is preferably prepared by mixing fluticasone propionate, albuterol sulfate and lactose. In the formulation of the present invention 99% w/w of the particles of fluticasone propionate and of albuterol sulfate each are less than 10 $\mu$m as measured by laser diffraction, preferably 90% w/w of the fluticasone propionate particles are less than 6 $\mu$m as measured by laser diffraction. And 90% w/w of the albuterol sulfate particles are smaller than 5 $\mu$m as measured by laser diffraction.

The particle sizes (mass median aerodynamic diameter, MMAD) of the fluticasone propionate used within the process of the present invention are less than 10 $\mu$m in size, more preferably 1-4 $\mu$m, and most preferably less than 3 $\mu$m. MMAD may be measured using a next generation impactor (NGI).

The particle sizes (mass median aerodynamic diameter, MMAD) of the albuterol sulfate used within the process of the present invention are less than 10 $\mu$m in size, more preferably 1-4 $\mu$m and most preferably less than 2 $\mu$m. MMAD may be measured using a next generation impactor (NGI).

This particle size ensures that the particles effectively adhere to the carrier during mixing, and also that the particles disperse and become entrained in the air stream and deposited in the lower lung (i.e. upon actuation of an inhaler device).

The dry powder formulation may be metered and filled into capsules, e.g. gelatin or hydroxypropyl methylcellulose capsules, such that the capsule contains a unit dose of active ingredient. When the dry powder is in a capsule containing a unit dose of active ingredient, the total amount of composition will depend on the size of the capsules and the characteristics of the inhalation device with which the capsules are being used. However, typical examples of total fill weights of dry powder per capsule are 1-25 mg. The formulation may also be filled into blister strips. Alternatively, the dry powder composition according to the invention may be filled into the reservoir of a multi-dose dry powder inhaler (MDPI).

Preferably, the multi-dose dry powder inhaler includes a cyclone deagglomerator for breaking up agglomerates of the active ingredients and carrier. This occurs prior to inhalation of the powder by a patient. The deagglomerator includes an inner wall defining a swirl chamber extending along an axis from a first end to a second end, a dry powder supply port, two inlet ports, and an outlet port.

The supply port is in the first end of the swirl chamber for providing fluid communication between a dry powder delivery passageway of the inhaler and the first end of the swirl chamber. The inlet port is in the inner wall of the swirl chamber adjacent to the first end of the swirl chamber and provides fluid communication between a region exterior to the deagglomerator and the swirl chamber. The outlet port provides fluid communication between the second end of the swirl chamber and a region exterior to the deagglomerator.

A breath induced low pressure at the outlet port causes air flows into the swirl chamber through the dry powder supply port and the inlet port. The air flows collide with each other and with the wall of the swirl chamber prior to exiting through the outlet port, such that the active is detached from the carrier (lactose). The deagglomerator further includes vanes at the first end of the swirl chamber for creating additional collisions and impacts of entrained powder.

A first breath-actuated air flow is directed for entraining a dry powder from an inhaler into a first end of a chamber extending longitudinally between the first end and a second end, the first air flow directed in a longitudinal direction.

A second breath-actuated airflow is directed in a substantially transverse direction into the first end of the chamber such that the air flows collide and substantially combine.

Then, a portion of the combined air flows is deflected in a substantially longitudinal direction towards a second end of the chamber, and a remaining portion of the combined air flows is directed in a spiral path towards the second end of the chamber. All the combined air flows and any dry powder entrained therein are then delivered from the second end of the chamber to a patient's mouth.

The deagglomerator ensures that particles of the actives are small enough for adequate penetration of the powder into a bronchial region of a patient's lungs during inhalation by the patient.

Thus, preferably, where the dry powder formulation of the present invention is used in conjunction with a multi-dose dry powder inhaler device, the deagglomerator of the inhaler device comprises: an inner wall defining a swirl chamber extending along an axis from a first end to a second end; a dry powder supply port in the first end of the swirl chamber for providing fluid communication between a dry powder delivery passageway of the inhaler and the first end of the swirl chamber; at least one inlet port in the inner wall of the swirl chamber adjacent to the first end of the swirl chamber providing fluid communication between a region exterior to the deagglomerator and the first end of the swirl chamber; an outlet port providing fluid communication between the second end of the swirl chamber and a region exterior to the deagglomerator; and vanes at the first end of the swirl chamber extending at least in part radially outwardly from the axis of the chamber, each of the vanes having an oblique surface facing at least in part in a direction transverse to the axis; whereby a breath induced low pressure at the outlet port causes air flows into the swirl chamber through the dry powder supply port and the inlet port.

In a preferred embodiment, the flow rate of the inhaler at a 4 kPa pressure drop is 59-71 L/min, most preferably 63 L/min. The flow resistance is preferably 0.028-0.034 KPa$^{0.5}$/L/min.

The inhaler preferably has a reservoir for containing the formulation and an arrangement for delivering a metered dose of the formulation from the reservoir. The reservoir is typically a pressure system. The inhaler preferably includes: a sealed reservoir including a dispensing port; a channel communicating with the dispensing port and including a pressure relief port; a conduit providing fluid communication between an interior of the sealed reservoir and the pressure relief port of the channel; and a cup assembly movably received in the channel and including, a recess adapted to receive formulation when aligned with the dispensing port, a first sealing surface adapted to seal the dispensing port when the recess is unaligned with the dispensing port, and a second sealing surface adapted to sealing the pressure relief port when the recess is aligned with the dispensing port and unseal the pressure relief port when the recess is unaligned with the dispensing port.

The inhaler preferably has a dose counter. The inhaler includes a mouthpiece for patient inhalation, a dose-metering arrangement including a pawl movable along a predetermined path during the metering of a dose of formulation to the mouthpiece by the dose-metering arrangement, and a dose counter.

In a preferred form, the dose counter includes a bobbin, a rotatable spool, and a rolled ribbon received on the bobbin, rotatable about an axis of the bobbin. The ribbon has indicia thereon successively extending between a first end of the ribbon secured to the spool and a second end of the ribbon positioned on the bobbin. The dose counter also includes teeth extending radially outwardly from the spool into the predetermined path of the pawl so that the spool is rotated by the pawl and the ribbon advanced onto the spool during the metering of a dose to the mouthpiece.

The preferred inhaler includes a simple, accurate and consistent mechanical dose metering system that dispenses dry powdered formulation in discrete amounts or doses for patient inhalation, a reservoir pressure system that ensures consistently dispensed doses, and a dose counter indicating the number of doses remaining in the inhaler.

The inhaler used in the present invention may also have electronic connectivity. This provides an inhalation monitoring system comprising: an inhaler comprising a medicament delivery apparatus configured to deliver the fixed-dose product of the present invention to a user during an inhalation of the user; an inhalation monitoring apparatus configured to, during said inhalation, gather data for determining a measure of the user's lung function and/or lung health and/or inhalation technique; and a processor configured to receive said data from said inhalation monitoring apparatus and, using the data, determine a measure of the user's lung function and/or lung health and/or inhalation technique. Further details may be found in WO 2016/090260 and WO 2020/222146.

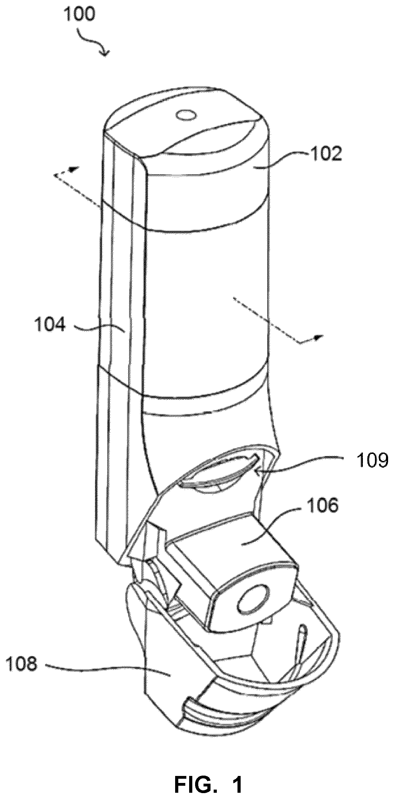

Figs. 1-4 provide views of an inhaler 100 for administering a dry powder inhalation composition according to any of the embodiments described herein.

The inhaler 100 may include a top cap 102, a main housing 104, a mouthpiece 106, a mouthpiece cover 108, an air vent 109, and an electronics module 120. The mouthpiece cover 108 may be hinged to the main housing 104 so that it may open and close to expose the mouthpiece 106. Although illustrated as a hinged connection, the mouthpiece cover 106 may be connected to the inhaler 100 through other types of connections. Moreover, while the electronics module 120 is illustrated as housed within the top cap 102 at the top of the main housing 104, the electronics module 120 may be integrated and/or housed within the main body 104 of the inhaler 100.

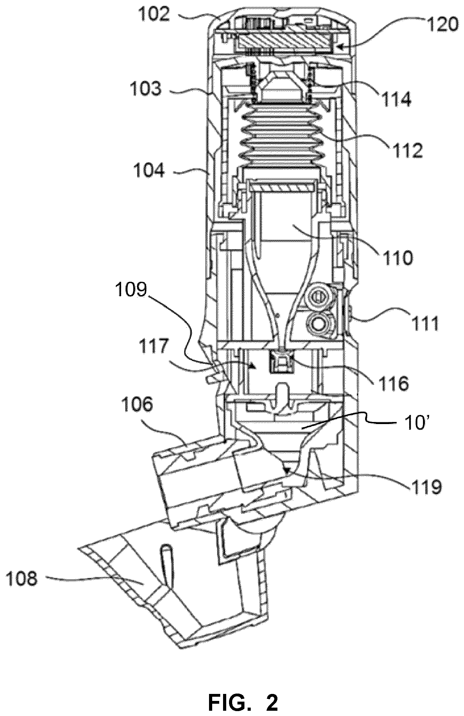

Fig. 2 provides a cross-sectional interior perspective view of the example inhaler 100. Inside the main housing 104, the inhaler 100 may include a medication reservoir 110 (e.g. a hopper), a bellows 112, a bellows spring 114, a yoke (not visible), a dosing cup 116, a dosing chamber 117, a deagglomerator 10', and a flow pathway 119. The medication reservoir 110 may include medication, in particular the dry powder inhalation composition, for delivery to the subject. When the mouthpiece cover 108 is moved from the closed to the open position, the bellows 112 may compress to deliver a dose of medication from the medication reservoir 110 to the dosing cup 116. Thereafter, a subject may inhale through the mouthpiece 106 in an effort to receive the dose of medication.

In a preferred embodiment, the dosing cup 116 has a capacity of 5-10 mm$^3$, and more preferably is a size 3 dose cup 116, which corresponds to a capacity of 7 mm$^3$.

The airflow generated from the subject's inhalation may cause the deagglomerator 10' to aerosolize the dose of medication by breaking down the agglomerates of the medicament in the dosing cup 116. The deagglomerator 10' may be configured to aerosolize the medication when the airflow through the flow pathway 119 meets or exceeds a particular rate, or is within a specific range. When aerosolized, the dose of medication may travel from the dosing cup 116, into the dosing chamber 117, through the flow pathway 119, and out of the mouthpiece 106 to the subject. If the airflow through the flow pathway 119 does not meet or exceed a particular rate, or is not within a specific range, the medication may remain in the dosing cup 116. In the event that the medication in the dosing cup 116 has not been aerosolized by the deagglomerator 10', another dose of medication may not be delivered from the medication reservoir 110 when the mouthpiece cover 108 is subsequently opened. Thus, a single dose of medication may remain in the dosing cup until the dose has been aerosolized by the deagglomerator 10'.

As the subject inhales through the mouthpiece 106, air may enter the air vent 109 to provide a flow of air for delivery of the medication to the subject. The flow pathway 119 may extend from the dosing chamber 117 to the end of the mouthpiece 106, and include the dosing chamber 117 and the internal portions of the mouthpiece 106. The dosing cup 116 may reside within or adjacent to the dosing chamber 117. Further, the inhaler 100 may include a dose counter 111 that is configured to be initially set to a number of total doses of medication within the medication reservoir 110 and to decrease by one each time the mouthpiece cover 108 is moved from the open position to the closed position.

When a dose of medication is delivered, a dose confirmation may be stored in a memory included in the electronics module 120 as dose confirmation information.

The top cap 102 may be attached to the main housing 104. For example, the top cap 102 may be attached to the main housing 104 through the use of one or more clips that engage recesses on the main housing 104. The top cap 102 may overlap a portion of the main housing 104 when connected, for example, such that a substantially pneumatic seal 103 exists between the top cap 102 and the main housing 104.

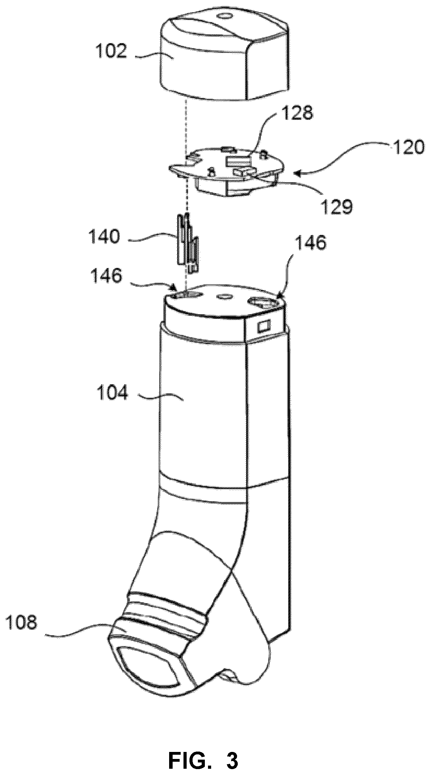

Fig. 3 is an exploded perspective view of the example inhaler 100 with the top cap 102 removed to expose the electronics module 120. As shown in Fig. 3, the top surface of the main housing 104 may include one or more (e.g. two) orifices 146. One or more (e.g. both) of the orifices 146 may be configured to accept a slider 140. For example, when the top cap 102 is attached to the main housing 104, the slider 140 may protrude through the top surface of the main housing 104 via one of the orifices 146.

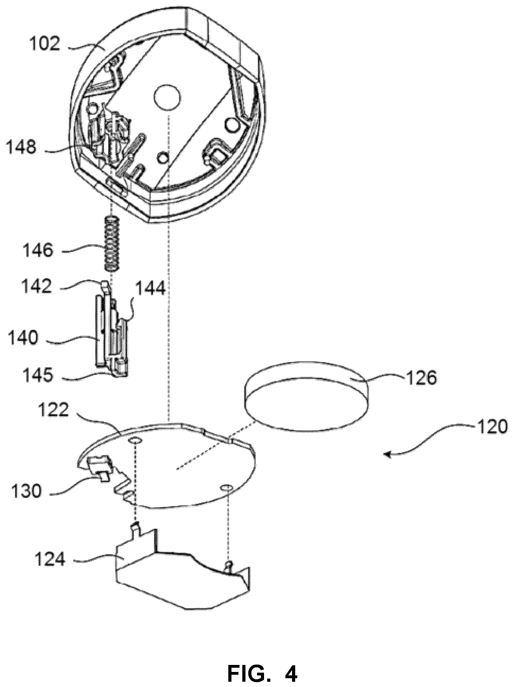

Fig. 4 is an exploded perspective view of the top cap 102 and the electronics module 120 of the example inhaler 100. As shown in Fig. 4, the slider 140 may define an arm 142, a stopper 144, and a distal end 145. The distal end 145 may be a bottom portion of the slider 140. The distal end 145 of the slider 140 may be configured to abut the yoke that resides within the main housing 104 (e.g. when the mouthpiece cover 108 is in the closed or partially open position). The distal end 145 may be configured to abut a top surface of the yoke when the yoke is in any radial orientation. For example, the top surface of the yoke may include a plurality of apertures (not shown), and the distal end 145 of the slider 140 may be configured to abut the top surface of the yoke, for example, whether or not one of the apertures is in alignment with the slider 140.

The top cap 102 may include a slider guide 148 that is configured to receive a slider spring 146 and the slider 140. The slider spring 146 may reside within the slider guide 148. The slider spring 146 may engage an inner surface of the top cap 102, and the slider spring 146 may engage (e.g. abut) an upper portion (e.g. a proximate end) of the slider 140. When the slider 140 is installed within the slider guide 148, the slider spring 146 may be partially compressed between the top of the slider 140 and the inner surface of the top cap 102. For example, the slider spring 146 may be configured such that the distal end 145 of the slider 140 remains in contact with the yoke when the mouthpiece cover 108 is closed. The distal end 145 of the slider 145 may also remain in contact with the yoke while the mouthpiece cover 108 is being opened or closed. The stopper 144 of the slider 140 may engage a stopper of the slider guide 148, for example, such that the slider 140 is retained within the slider guide 148 through the opening and closing of the mouthpiece cover 108, and vice versa. The stopper 144 and the slider guide 148 may be configured to limit the vertical (e.g. axial) travel of the slider 140. This limit may be less than the vertical travel of the yoke. Thus, as the mouthpiece cover 108 is moved to a fully open position, the yoke may continue to move in a vertical direction towards the mouthpiece 106 but the stopper 144 may stop the vertical travel of the slider 140 such that the distal end 145 of the slider 140 may no longer be in contact with the yoke.

More generally, the yoke may be mechanically connected to the mouthpiece cover 108 and configured to move to compress the bellows spring 114 as the mouthpiece cover 108 is opened from the closed position and then release the compressed bellows spring 114 when the mouthpiece cover reaches the fully open position, thereby causing the bellows 112 to deliver the dose from the medication reservoir 110 to the dosing cup 116. The yoke may be in contact with the slider 140 when the mouthpiece cover 108 is in the closed position. The slider 140 may be arranged to be moved by the yoke as the mouthpiece cover 108 is opened from the closed position and separated from the yoke when the mouthpiece cover 108 reaches the fully open position.

The movement of the slider 140 during the dose metering may cause the slider 140 to engage and actuate a switch 130. The switch 130 may trigger the electronics module 120 to register the dose metering. The slider 140 may be regarded in this example as the means by which a use determination system is configured to register the metering of the dose by the dose metering system, each metering being thereby indicative of an inhalation performed by the subject using the inhaler 100.

Actuation of the switch 130 by the slider 140 may also, for example, cause the electronics module 120 to transition from the first power state to a second power state, and to sense an inhalation by the subject from the mouthpiece 106.

The electronics module 120 may include a printed circuit board (PCB) assembly 122, a switch 130, a power supply (e.g. a battery 126), and/or a battery holder 124. Referring to Figs. 3 and 4, the PCB assembly 122 may include surface mounted components, such as a sensor system 128, a wireless communication circuit 129, the switch 130, and/or one or more indicators (not shown), such as one or more light emitting diodes (LEDs). The electronics module 120 may include a controller (e.g. a processor) and/or memory. The controller and/or memory may be physically distinct components of the PCB assembly 122. Alternatively, the controller and memory may be part of another chipset mounted on the PCB assembly 122, for example, the wireless communication circuit 129 may include the controller and/or memory for the electronics module 120. The controller of the electronics module 120 may include a microcontroller, a programmable logic device (PLD), a microprocessor, an application specific integrated circuit (ASIC), a field programmable gate array (FPGA), or any suitable processing device or control circuit.

The controller may access information from, and store data in the memory. The memory may include any type of suitable memory, such as non-removable memory and/or removable memory. The non-removable memory may include random-access memory (RAM), read-only memory (ROM), or any other type of memory storage device. The removable memory may include a subscriber identity module (SIM) card, a memory stick, a secure digital (SD) memory card, and the like. The memory may be internal to the controller. The controller may also access data from, and store data in, memory that is not physically located within the electronics module 120, such as on a server or a smart phone.

The sensor system 128 may include one or more sensors. The sensor system 128 may include one or more sensors, for example, of different types, such as, but not limited to one or more pressure sensors, temperature sensors, humidity sensors, orientation sensors, acoustic sensors, and/or optical sensors. The one or more pressure sensors may include a barometric pressure sensor (e.g. an atmospheric pressure sensor), a differential pressure sensor, an absolute pressure sensor, and/or the like. The sensors may employ microelectromechanical systems (MEMS) and/or nanoelectromechanical systems (NEMS) technology. The sensor system 128 may be configured to provide an instantaneous reading (e.g. pressure reading) to the controller of the electronics module 120 and/or aggregated readings (e.g. pressure readings) overtime. As illustrated in Figs. 2 and 3, the sensor system 128 may reside outside the flow pathway 119 of the inhaler 100, but may be pneumatically coupled to the flow pathway 119.

The controller of the electronics module 120 may receive signals corresponding to measurements from the sensor system 128. The controller may calculate or determine one or more airflow metrics using the signals received from the sensor system 128. The airflow metrics may be indicative of a profile of airflow through the flow pathway 119 of the inhaler 100. For example, if the sensor system 128 records a change in pressure of 0.3 kilopascals (kPa), the electronics module 120 may determine that the change corresponds to an airflow rate of approximately 45 liters per minute (Lpm) through the flow pathway 119.

The pressure measurement readings and/or the computed airflow metrics may be indicative of the quality or strength of inhalation from the inhaler 100. For example, when compared to a particular threshold or range of values, the readings and/or metrics may be used to categorize the inhalation as a certain type of event, such as a good inhalation event, a low inhalation event, a no inhalation event, or an excessive inhalation event. The categorization of the inhalation may be usage parameters stored as personalized data of the subject.

The no inhalation event may be associated with pressure measurement readings and/or airflow metrics below a particular threshold, such as an airflow rate less than 30 Lpm. The no inhalation event may occur when a subject does not inhale from the mouthpiece 106 after opening the mouthpiece cover 108 and during the measurement cycle. The no inhalation event may also occur when the subject's inspiratory effort is insufficient to ensure proper delivery of the medication via the flow pathway 119, such as when the inspiratory effort generates insufficient airflow to activate the deagglomerator 10' and, thus, aerosolize the medication in the dosing cup 116.

The low inhalation event may be associated with pressure measurement readings and/or airflow metrics within a particular range, such as an airflow rate between 30 Lpm and 45 Lpm. The low inhalation event may occur when the subject inhales from the mouthpiece 106 after opening the mouthpiece cover 108 and the subject's inspiratory effort causes at least a partial dose of the medication to be delivered via the flow pathway 119. That is, the inhalation may be sufficient to activate the deagglomerator 10' such that at least a portion of the medication is aerosolized from the dosing cup 116.

The good inhalation event may be associated with pressure measurement readings and/or airflow metrics above the low inhalation event, such as an airflow rate between 45 Lpm and 200 Lpm. The good inhalation event may occur when the subject inhales from the mouthpiece 106 after opening the mouthpiece cover 108 and the subject's inspiratory effort is sufficient to ensure proper delivery of the medication via the flow pathway 119, such as when the inspiratory effort generates sufficient airflow to activate the deagglomerator 10' and aerosolize a full dose of medication in the dosing cup 116.

The excessive inhalation event may be associated with pressure measurement readings and/or airflow metrics above the good inhalation event, such as an airflow rate above 200 Lpm. The excessive inhalation event may occur when the subject's inspiratory effort exceeds the normal operational parameters of the inhaler 100. The excessive inhalation event may also occur if the inhaler 100 is not properly positioned or held during use, even if the subject's inspiratory effort is within a normal range. For example, the computed airflow rate may exceed 200 Lpm if the air vent 109 is blocked or obstructed (e.g. by a finger or thumb) while the subject is inhaling from the mouthpiece 106.

Any suitable thresholds or ranges may be used to categorize a particular event. Some or all of the events may be used. For example, the no inhalation event may be associated with an airflow rate below 45 Lpm and the good inhalation event may be associated with an airflow rate between 45 Lpm and 200 Lpm. As such, the low inhalation event may not be used at all in some cases.

The pressure measurement readings and/or the computed airflow metrics may also be indicative of the direction of flow through the flow pathway 119 of the inhaler 100. For example, if the pressure measurement readings reflect a negative change in pressure, the readings may be indicative of air flowing out of the mouthpiece 106 via the flow pathway 119. If the pressure measurement readings reflect a positive change in pressure, the readings may be indicative of air flowing into the mouthpiece 106 via the flow pathway 119. Accordingly, the pressure measurement readings and/or airflow metrics may be used to determine whether a subject is exhaling into the mouthpiece 106, which may signal that the subject is not using the inhaler 100 properly.

The personalized data collected from, or calculated based on, the usage of the inhaler 100 (e.g. pressure metrics, airflow metrics, lung function metrics, dose confirmation information, etc.) may be computed and/or assessed via external devices as well (e.g. partially or entirely). More specifically, the wireless communication circuit 129 in the electronics module 120 may include a transmitter and/or receiver (e.g. a transceiver), as well as additional circuity. For example, the wireless communication circuit 129 may include a Bluetooth chip set (e.g. a Bluetooth Low Energy chip set), a ZigBee chipset, a Thread chipset, etc. As such, the electronics module 120 may wirelessly provide the personalized data, such as pressure measurements, airflow metrics, lung function metrics, dose confirmation information, and/or other conditions related to usage of the inhaler 100, to an external device, including a smart phone. The personalized data may be provided in real time to the external device to enable an assessment of a status of the subject's respiratory disease to be generated based on real-time data from the inhaler 100 that indicates time of use, how the inhaler 100 is being used, and personalized data about the user of the inhaler, such as real-time data related to the subject's lung function and/or medical treatment. The external device may include software for processing the received information and for providing compliance and adherence feedback to users of the inhaler 100 via a graphical user interface (GUI).

The airflow metrics may include personalized data that is collected from the inhaler 100 in real-time, such as one or more of an average flow of an inhalation/exhalation, a peak flow of an inhalation/exhalation (e.g. a maximum inhalation received), a volume of an inhalation/exhalation, a time to peak of an inhalation/exhalation, and/or the duration of an inhalation/exhalation. The airflow metrics may also be indicative of the direction of flow through the flow pathway 119. That is, a negative change in pressure may correspond to an inhalation from the mouthpiece 106, while a positive change in pressure may correspond to an exhalation into the mouthpiece 106. When calculating the airflow metrics, the electronics module 120 may be configured to eliminate or minimize any distortions caused by environmental conditions. For example, the electronics module 120 may re-zero to account for changes in atmospheric pressure before or after calculating the airflow metrics. The one or more pressure measurements and/or airflow metrics may be timestamped and stored in the memory of the electronics module 120.

In addition to the airflow metrics, the inhaler 100, or another computing device, may use the airflow metrics to generate additional personalized data. For example, the controller of the electronics module 120 of the inhaler 100 may translate the airflow metrics into other metrics that indicate the subject's lung function and/or lung health that are understood to medical practitioners, such as peak inspiratory flow metrics, peak expiratory flow metrics, and/or forced expiratory volume in 1 second (FEV1), for example. The electronics module 120 of the inhaler may determine a measure of the subject's lung function and/or lung health using a mathematical model such as a regression model. The mathematical model may identify a correlation between the total volume of an inhalation and FEV1. The mathematical model may identify a correlation between peak inspiratory flow and FEV1. The mathematical model may identify a correlation between the total volume of an inhalation and peak expiratory flow. The mathematical model may identify a correlation between peak inspiratory flow and peak expiratory flow.

The battery 126 may provide power to the components of the PCB assembly 122. The battery 126 may be any suitable source for powering the electronics module 120, such as a coin cell battery, for example. The battery 126 may be rechargeable or non-rechargeable. The battery 126 may be housed by the battery holder 124. The battery holder 124 may be secured to the PCB assembly 122 such that the battery 126 maintains continuous contact with the PCB assembly 122 and/or is in electrical connection with the components of the PCB assembly 122. The battery 126 may have a particular battery capacity that may affect the life of the battery 126. As will be further discussed below, the distribution of power from the battery 126 to the one or more components of the PCB assembly 122 may be managed to ensure the battery 126 can power the electronics module 120 over the useful life of the inhaler 100 and/or the medication contained therein.

In a connected state, the communication circuit and memory may be powered on and the electronics module 120 may be "paired" with an external device, such as a smart phone. The controller may retrieve data from the memory and wirelessly transmit the data to the external device. The controller may retrieve and transmit the data currently stored in the memory. The controller may also retrieve and transmit a portion of the data currently stored in the memory. For example, the controller may be able to determine which portions have already been transmitted to the external device and then transmit the portion(s) that have not been previously transmitted. Alternatively, the external device may request specific data from the controller, such as any data that has been collected by the electronics module 120 after a particular time or after the last transmission to the external device. The controller may retrieve the specific data, if any, from the memory and transmit the specific data to the external device.

The data stored in the memory of the electronics module 120 (e.g. the signals generated by the switch 130, the pressure measurement readings taken by the sensory system 128 and/or the airflow metrics computed by the controller of the PCB assembly 122) may be transmitted to an external device, which may process and analyze the data to determine usage parameters associated with the inhaler 100. Further, a mobile application residing on the mobile device may generate feedback for the user based on data received from the electronics module 120. For example, the mobile application may generate daily, weekly, or monthly reports, provide confirmation of error events or notifications, provide instructive feedback to the subject, and/or the like.

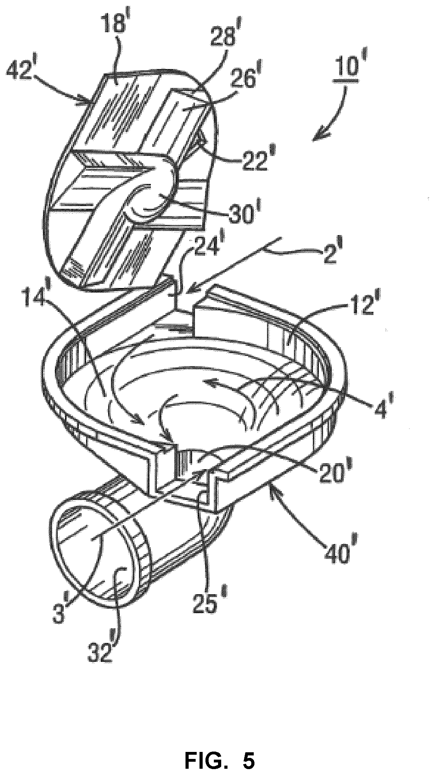

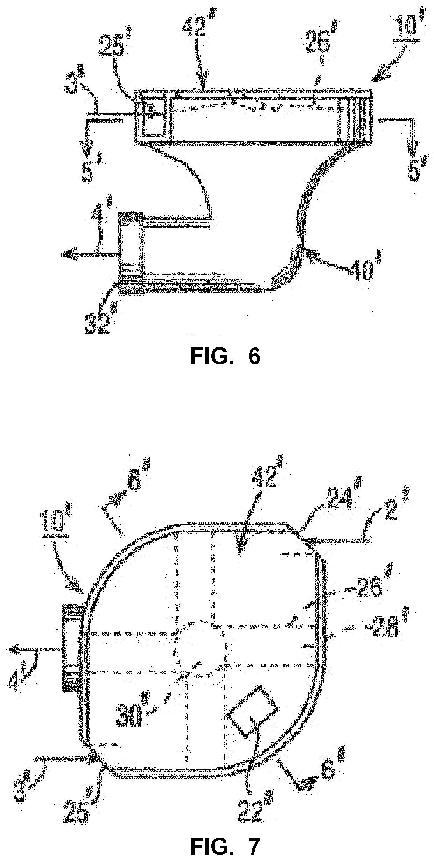

Referring to Figs. 5-10, the deagglomerator 10' breaks down agglomerates of dry powder formulation, or formulation and carrier, before the dry powder leaves the inhaler 100 through the mouthpiece 106. Thus, the agglomerates of dry powder formulation, or formulation and carrier, are broken down by the deagglomerator 10' before inhalation of the formulation by a patient.

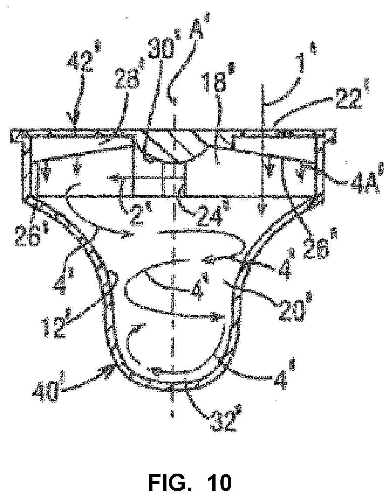

In general, the deagglomerator 10' includes an inner wall 12' defining a swirl chamber 14' extending along an axis A' (see Fig. 10) from a first end 18' to a second end 20'. The swirl chamber 14' includes circular cross-sectional areas arranged transverse to the axis A', that decrease from the first end 18' to the second end 20' of the swirl chamber 14', such that any air flow traveling from the first end of the swirl chamber to the second end is constricted and at least in part collide with the inner wall 12' of the chamber.

Preferably, the cross-sectional areas of the swirl chamber 14' decrease monotonically. In addition, the inner wall 12' is preferably convex, i.e. arches inwardly towards the axis A', as shown best in Fig. 10.

As shown in Figs. 5, 7 and 10, the deagglomerator 10' also includes a dry powder supply port 22' in the first end 18' of the swirl chamber 14' for providing fluid communication between a dry powder delivery passageway of an inhaler and the first end 18' of the swirl chamber 14'. Preferably, the dry powder supply port 22' faces in a direction substantially parallel with the axis A' such that an air flow, illustrated by arrow 1' in Fig. 10, entering the chamber 14' through the supply port 22' is at least initially directed parallel with respect to the axis A' of the chamber.

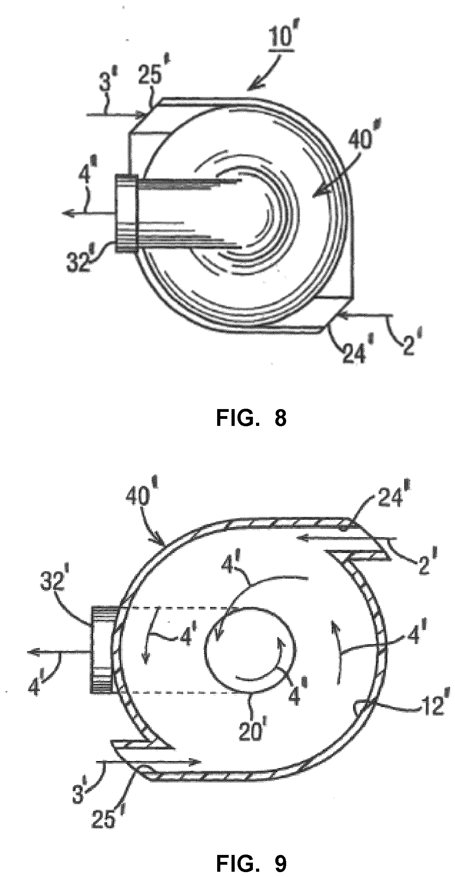

Referring to Figs. 5-10, the deagglomerator 10' additionally includes at least one inlet port 24' in the inner wall 12' of the swirl chamber 14' adjacent to or near the first end 18' of the chamber providing fluid communication between a region exterior to the deagglomerator and the first end 18' of the swirl chamber 14'. Preferably, the at least one inlet port comprises two diametrically opposed inlet ports 24', 25' that extend in a direction substantially transverse to the axis A' and substantially tangential to the circular cross-section of the swirl chamber 14'. As a result, air flows, illustrated by arrows 2' and 3' in Figs. 5 and 9, entering the chamber 14' through the inlet ports are at least initially directed transverse with respect to the axis A' of the chamber and collide with the air flow 1' entering through the supply port 22' to create turbulence. The combined air flows, illustrated by arrow 4' in Figs. 9 and 10, then collide with the inner wall 12' of the chamber 14', form a vortex, and create additional turbulence as they move towards the second end 20' of the chamber.

Referring to Figs. 5-7 and 10, the deagglomerator 10' includes vanes 26' at the first end 18' of the swirl chamber 14' extending at least in part radially outwardly from the axis A' of the chamber. Each of the vanes 26' has an oblique surface 28' facing at least in part in a direction transverse to the axis A' of the chamber. The vanes 26' are sized such that at least a portion 4A' of the combined air flows 4' collide with the oblique surfaces 28', as shown in Fig. 10. Preferably, the vanes comprise four vanes 26', each extending between a hub 30' aligned with the axis A' and the wall 12' of the swirl chamber 14'.

As shown in Figs. 5-10, the deagglomerator 10' further includes an outlet port 32' providing fluid communication between the second end 20' of the swirl chamber 14' and a region exterior to the deagglomerator. A breath induced low pressure at the outlet port 32' causes the air flow 1' through the supply port 22' and the air flows 2',3' through the inlet ports and draws the combined air flow 4' through the swirl chamber 14'. The combined air flow 4' then exits the deagglomerator through the outlet port 32'. Preferably the outlet port 32' extends substantially transverse to the axis A', such that the air flow 4' will collide with an inner wall of the outlet port 32' and create further turbulence. The term "substantially transverse" is intended to cover the outlet port 32' extending perpendicularly, in other words at 90 degrees, with respect to the axis A', but also the outlet port 32' extending at other angles with respect to the axis A', provided that the above-mentioned further turbulence can be created via collision of the air flow 4' with the outlet port's 32' inner wall. For example, the outlet port 32' may extend at an angle of about 15 degrees with respect to a perpendicular of the axis A'.

During use of the deagglomerator 10' in combination with the inhaler, patient inhalation at the outlet port 32' causes air flows 1',2',3' to enter through, respectively, the dry powder supply port 22' and the inlet ports. Although not shown, the air flow 1' through the supply port 22' entrains the dry powder into the swirl chamber 14'. The air flow 1' and entrained dry powder are directed by the supply port 22' into the chamber in a longitudinal direction, while the air flows 2',3' from the inlet ports are directed in a transverse direction, such that the air flows collide and substantial combine.

A portion of the combined air flow 4' and the entrained dry powder then collide with the oblique surfaces 28' of the vanes 26' causing particles and any agglomerates of the dry powder to impact against the oblique surfaces and collide with each other. The geometry of the swirl chamber 14' causes the combined air flow 4' and the entrained dry powder to follow a turbulent, spiral path, or vortex, through the chamber. As will be appreciated, the decreasing cross-sections of the swirl chamber 14' continuously changes the direction and increases the velocity of the spiraling combined air flow 4' and entrained dry powder. Thus, particles and any agglomerates of the dry powder constantly impact against the wall 12' of the swirl chamber 14' and collide with each other, resulting in a mutual grinding or shattering action between the particles and agglomerates. In addition, particles and agglomerates deflected off the oblique surfaces 28' of the vanes 26' cause further impacts and collisions.

Upon exiting the swirl chamber 14', the direction of the combined air flow 4 and the entrained dry powder is again changed to a transverse direction with respect to the axis A', through the outlet port 32'. The combined air flow 4' and the entrained dry powder retain a swirl component of the flow, such that the air flow 4' and the entrained dry powder spirally swirls through the outlet port 32'. The swirling flow causes additional impacts in the outlet port 32' so as to result in further breaking up of any remaining agglomerates prior to being inhaled by a patient.

As shown in Figs. 5-10, the deagglomerator is preferably assembled from two pieces: a cup-like base 40' and a cover 42'. The base 40' and the cover 42' are connected to form the swirl chamber 14'. The cup-like base 40' includes the wall 12' and the second end 20' of the chamber and defines the outlet port 32'. The base 40' also includes the inlet ports of the swirl chamber 14'. The cover 42' forms the vanes 26' and defines the supply port 22'.

The base 40' and the cover 42' of the deagglomerator are preferably manufactured from a plastic such as ABS for the base and polypropylene for the cover, but may be manufactured from metal or another suitable material. The base 40' and the cover 42' can be connected to each other in any suitable manner, for example by the base 40' being ultrasonically welded to the spacer 38', and the cover 42' being push-fitted into the underside of the spacer 38' and held in place.

The inhaler 100 shown in Figs. 1-4 may include the particular deagglomerator 10' described with reference to Figs. 5-10. However, the inhaler 100 is not limited to use with the deagglomerator shown in Figs. 1-4 and can be used with other types of deagglomerators or a simple swirl chamber.

It is also noted that the inhaler 100 having electronic connectivity is not intended to be limiting. In this respect, the present disclosure contemplates the inhaler 100 having the basic mechanical structure described above with reference to Figs. 1-10, or the mechanical structure described in US 2015/099726 A1, but with the electronics module 120 being omitted.

Regarding the mechanical structure of the inhaler and/or the deagglomerator, the disclosure of US 2015/099726 A1 is incorporated by reference in its entirety.

To the extent that any meaning or definition of a term in this document conflicts with any meaning or definition of the same term in a document incorporated by reference, such as US 2015/099726 A1, the meaning or definition assigned to that term in this document shall govern.

The present invention will now be described with reference to the following examples which are not intended to be limiting.

**Examples**

**Example 1 - fixed dose fluticasone propionate and albuterol sulfate formulations**

55 mcg FP /117mcg ABS

0.1760 kg Fluticasone propionate (Fp) was blended together with 7.8240 kg of $\alpha$-lactose monohydrate carrier for 10 min at 433 rpm (revolutions per minute) using a PharmaConnect high shear blender providing an 8 kg blend. 0.9400 kg of albuterol sulfate (ABS) was blended together with 19.0600 kg of $\alpha$-lactose monohydrate carrier for 7 min at 300 rpm using a PharmaConnect high shear blender providing 20 kg of a blend. 8 kg of the Fp-containing blend and 8 kg of the ABS-containing blend were then mixed together using PharmaConnect low shear blender and mixed at 15 rpm for 20 mins to provide a final combination blend containing 1.1% w/w of fluticasone propionate (suitable for providing a 51 $\mu$g dose, size 3 dose cup) and 2.35% w/w of albuterol sulfate (suitable for providing a 90 $\mu$g albuterol base per dose, size 3 dose cup). The final combination blend was then filled into the reservoir of a dry powder inhaler device. The devices were subjected to 30°C/65%RH for 4 weeks for conditioning and then packed in a foil overwrap with a desiccant.

30 mcg FP /117mcg ABS

0.0873 kg of fluticasone propionate (Fp) was blended together with 7.9127 kg of $\alpha$-lactose monohydrate carrier for 12 min at 150 rpm using a PharmaConnect high shear Blender providing 8 kg of a blend. 0.9400 kg of albuterol sulfate (ABS) was blended together with 19.0600 kg of $\alpha$-lactose monohydrate carrier for 7 min at 300 rpm using a Pharma-Connect high shear blender providing 20 kg of a blend. 8 kg of the Fp-containing blend and 8 kg of the ABS-containing blend were then mixed together using PharmaConnect low shear blender and mixed at 15 rpm for 20 min to provide a final combination blend containing 0.55% w/w of fluticasone propionate (suitable for providing a 26 $\mu$g dose, size 3 dose cup) and 2.35% w/w of albuterol sulfate (suitable for providing a 90 $\mu$g albuterol base per dose, size 3 dose cup). The final combination blend was then filled into the reservoir of a dry powder inhaler device. The devices were subjected to 30°C/65%RH for 4 weeks for conditioning and then packed in a foil overwrap with a desiccant.

**Example 2 - analytical methods**

Albuterol sulfate PSD measurement

Test Method for Particle Size (Sympatec)
Operating Conditions for the RODOS M Dry Powder Disperser and Aspiros
Dispersion Method:
Air Pressure: 3.0 Bar
Feeder: Aspiros
Feed velocity/ rate: 18 mm/s
Lens: R3
Channel: N/A
Trigger Condition
Start: c. optical > 2%
Valid: Always
Stop: 2s c. optical ≤ 2% or 5 seconds real time
Time Base: 2.0 ms

Approximately 50 mg of the dry powder ABS was weighed into Aspiros test tube following by inserting the tube into the Aspiros and running the method. This was then blown by compressed air via the RODOS M dry powder dispenser through the measuring zone triggering a measurement.

Fluticasone propionate PSD measurement

Measuring Parameters:

Reagents:

De-ionized water Milli-Q
Tween 80 Reagent Grade
TA-10X FG defoamer N/A

Equipment:

Instrument: Malvern Laser Diffraction Mastersizer 2000
Accessory name: Hydro 2000s

Equipment Parameters:

Measuring Range: 0.02-2000 mcm
Model Type: General Purpose
Sensitivity: Normal
Particle Shape: Irregular
Mode: Wet Dispersion
Dispersant name: De-ionized water
Dispersant RI: 1.33
Material RI: 1.53
Absorption: 3.0
Measuring time: 10 seconds (10,000 snaps).
Background measuring time: 10 seconds (10,000 snaps).
Speed/stir speed: 2,000 rpm.
Aliquots: 1

Measurements: 3 per Aliquot.
Delay: 0 seconds between measurements.
Ultrasonic level: 100%
Obscuration: 10-30%
Measuring Procedure

Sample Preparation for Wet Measurement:

Preparation of 1% (W/V) Tween 80: 30mL of de-ionized water were added into a clean 50 mL glass bottle containing a magnetic stirring bar. 0.3 g of Tween 80 was added to the glass vessel and then stirred for about 10 minutes until a homogeneous solution was obtained.

Preparation of 1% (W/V) TA-10X FG defoamer: 10 mL of de-ionized water was added into a clean 25 mL glass vial. Add 0.1 g of TA-10X FG defoamer. The vial was tightly closed with cap, then hand-shaken for about 30 seconds. The obtained emulsion was shaken for a couple seconds immediately before each usage.

Preparation for sampling: Approximately 1-10 g of sample was transferred into a glass bottle large enough so that the powder was freely tumbled to assure a homogenous sample throughout the bottle. The capped bottle was gently inverted up and down 10 times, and then rotated 10 times in a clockwise manner.

Preparation of the sample: About 50 mg of the sample powder was transferred with a spatula into a clean 25 mL glass vessel containing a magnetic stirring bar. 10 mL of de-ionized water containing 1% Tween 80 were added into the glass vessel. The suspension was stirred with the magnetic stirrer for 2 minutes at a moderate speed. The sample was then ready to be loaded into the wet dispersion unit.

Manual measurement

The Hydro 2000S dispersion unit tank was manually filled with about 150 mL of de-ionized water. The ultrasonics was set at the level of 100% and sonicated for 30 seconds to allow air to dissipate, and then turned off. The pump/stirrer speed was turned up to full (3500 rpm), then down to zero to clear any bubbles.

About 0.3 mL of 1% TA-10X FG defoamer was added to the tank and then the pump/stirrer was resumed at 2000rpm. The background was measured by slowly dropping 1 out of 3 prepared samples from into the dispersion unit until a stabilized initial obscuration at 10-20% was reached. The sample was stirred in the dispersion unit for about 1 minute at 2,000 rpm and then turned on the ultrasound and the level was set to 100%. After about one minute the sample was measured and the result calculated.

<u>Lactose monohydrate PSD measurement</u>

Instrument: Sympatec HELOS/BR Particle size Analyzer. Reference SOPM000564
Ensure testing is performed using the PSD composite sample
Set up the following operating conditions using the Vibratory Feeder and Funnel Adaptor
Instrument Settings
Parameter Setting
Sympatec Dispersion method: 1.5bar Vibri 75%
Lens type: R4
Dispenser: RODOS M
Measuring Range: R4: 0.5/1.8...350 mcm
Trigger Conditions
Name: Ch. 9 optical conc. $\geq$ 0.5%
Reference duration: 4s (single)
Time base: 100 ms
Focus prior to first measurement: Yes
Normal measurement: Standard mode*
Start: 0.000s, channel. 9 optical conc. $\geq$ 0.5%
Valid: Always
Stop after 1 sec, channel. 9 optical conc. < 0.5%
Or after: 60.000s, real time
Repeat measurement: 0 times
Repeat focus: No

Disperser Conditions
Name: 1.5 bar; 75%; 2 mm
Dispersing type: RODOS/M
Injector: 4mm
With: 0 cascade elements
Primary pressure: 1.5 bar
Always auto adjust before reference measurement: No
Feeder type: VIBRI
Feed rate: 75%
Gap width: 2 mm
Funnel rotation: 0%
Cleaning time: 10s
Use VIBRI control: No
Vacuum extraction type: Nilfisk
Delay: 5s

The sample was mixed for 20 seconds by turning / rotating the sample. A suitable transfer container was used to weigh and transfer approximately 5.0 g of the sample from the composite container into the VIBRI funnel of the sample unit. This was then blown by compressed air via the RODOS dry powder dispenser through the measuring zone triggering a measurement.

**Example 3 - clinical trial**

This example sets out a 4-week, double-blind, placebo-controlled, parallel-group, efficacy and safety trial of fluticasone propionate/albuterol sulfate combination compared to fluticasone propionate, albuterol sulfate or placebo delivered by multidose dry powder inhaler in participants 12 years and older with asthma.

Rationale

This trial was designed to demonstrate the combination rule required by the Food and Drug Administration (FDA) according to 21 Code of Federal Regulations (CFR) §300.50(a) for this fixed dose combination (FDC) of fluticasone propionate/albuterol sulfate multidose dry powder inhaler with electronic module (Fp/ABS eMDPI).
The rationale for four times daily (QID) dosing in this trial is to examine safety and efficacy at the approximate duration of the bronchodilatory effect for albuterol, a component of the investigational medicinal product (IMP), Fp/ABS eMDPI. The trial design allows the "combination rule" to be evaluated, while providing safety data for chronic QID dosing of Fp/ABS eMDPI. In addition, it aims to provide demonstration of the bronchodilatory effect of the IMP at first exposure.

IMP= Fp/ABS (inhaled corticosteroid [ICS]/short-acting β-2 agonist [SABA]) eMDPI

General study design

This is a 4-week, double-blind, parallel-group, placebo-controlled randomized clinical trial to evaluate the efficacy and safety of Fp/ABS eMDPI 55/117 mcg in participants aged 12 years and older with asthma. The purpose of this trial is to compare Fp/ABS eMDPI 55/117 mcg to placebo and its constituent monotherapy components Fp eMDPI 55 mcg and ABS eMDPI 117 mcg on improvement in lung function over 4 weeks of treatment in participants aged 12 years and older with asthma.
In this trial we will compare Fp/ABS eMDPI 55/117 mcg to placebo and its constituent monotherapy components Fp eMDPI 55 mcg and ABS eMDPI 117 mcg testing the improvement in lung function over 4 weeks of treatment in participants aged 12 years and older with asthma. In addition, the trial will demonstrate the immediate effect on lung function at first dose and collect safety data on the FDC. Eligible participants are randomly assigned to receive 2 inhalations (1 dose) QID of either the IMP (Fp/ABS eMDPI 55/117 mcg), Fp eMDPI 55 mcg, ABS eMDPI 117 mcg, or placebo eMDPI for the 4 week treatment period. The trial consists of 3 periods after the screening period (up to 2 weeks, from Visit 1 (V1) to V2):

-   a single-blind placebo run-in period: From V2 to the baseline V3, 14-21 days (2-3 weeks);
-   double-blind treatment period: V3 through V5, approximately 28 days (4 weeks); and
-   a follow-up period: V6 approximately $7 \pm 3$ days after V5 (1 week).

The duration of participation is up to approximately 11 weeks including an optional prescreening visit.

Adverse events and concomitant medication inquiry occurs at each visit after informed consent/assent is signed as applicable.

Trial periods

Screening visit

The screening visit (V1) may occur up to approximately 14 days prior to V2 and on more than one day as needed to allow trial procedures to be completed. An informed consent/assent (as applicable) must be completed prior to any trial related procedures unless already completed at a prescreening visit.

Participants being treated with an ICS with or without a long acting $\beta_2$-agonist (LABA) with or without a non-corticosteroid (NCS) therapy are eligible to be included in the trial; participants may also have been treated with NCS therapy as their only treatment. Allowable NCS therapy include inhaled SABA alone or in combination with leukotriene modifiers, or theophylline. Participants are required to withhold inhaled asthma maintenance medications for approximately 24 hours prior to lung function testing for once daily medications or 12 hours for twice daily, or more frequently dosed, medication. If the participant has taken inhaled asthma maintenance medication within $24 \pm 2$ hours (daily dosed) or $12 \pm 2$ hours (dosed twice daily or more frequently) or SABA within 6 hours of the planned pulmonary function testing (PFT) or ICS/ LABA used as rescue medication within 12 hours of the planned pulmonary function testing, the visit must be rescheduled. Trial procedures including urine drug and pregnancy screening (women of childbearing potential), serum follicle-stimulating hormone (FSH) to confirm post-menopausal state as applicable, medical including asthma history, prior and concomitant medications and current asthma medications, demographics, a brief physical examination including vital signs are conducted during the screening period.

Pulmonary function testing and response to bronchodilator testing is assessed during the screening period. Participants may repeat the PFT including response to bronchodilator testing once during screening in order to qualify for inclusion. Historical response to bronchodilator (reversibility) data is not acceptable. In addition, the inclusion and exclusion criteria are to determine participants eligibility to enter the run-in period. Airway reversibility (response to bronchodilator testing), as measured by $FEV_1$, at screening is demonstrated after pre-albuterol pulmonary function testing is completed via central spirometry and then repeating $FEV_1$ measurement approximately 15 to 45 minutes after receiving 2 to 4 inhalations (based on investigator judgment) SABA medication. If a participants $FEV_1$ improves at least 12% between the 2 tests and an increase of 200 mL (for participants $\geq$18 years of age only) the participants are deemed to have met the response to bronchodilator requirement. Participants 12 to 17 do not need to meet the 200 mL requirement.

Run-in period

Participants meeting all of the selection criteria will enter the run-in period at V2. At this visit, participants are trained on proper inhaler technique (using the training inhaler), diary procedures and daily morning peak expiratory flow (PEF) measurements. Clinical laboratory testing is completed and vital signs are collected. The participants will discontinue all their current asthma maintenance and rescue medications for the remainder of the trial until V5 procedures have been completed if, in the investigator's judgement, there is no inherent harm in changing the participant's treatment regimen. They are supplied with single-blind placebo eMDPI to be taken as 2 inhalations (one dose) QID. The doses are to be separated over the course of the day, early morning, midday, early evening and prior to bedtime as much as possible and the participants should rinse their mouths with water without swallowing after each dose. Participants are supplied with albuterol (salbutamol) hydrofluoroalkane (HFA) inhalation aerosol (90 mcg ex-actuator or equivalent) pressurized metered dose inhaler (pMDI) as rescue medication. Participants may take 2 inhalations up to 6 times daily as needed to control asthma symptoms. Participants will continue in the run-in period for a minimum of 14 days but may extend to 21 days as needed to facilitate scheduling as required. Participants are instructed to complete the diary twice daily including morning PEF on the handheld device prior to single-blind inhaler use, daytime and nighttime asthma symptom scores, number of inhalations of rescue medication and confirm use of the single-blind inhaler at the various time points (morning, midday, early evening and bedtime).

Base-line visit

The baseline visit (V3, Day 1) is conducted at the end of the run-in period. Participants are evaluated based on trial requirements to determine if they can be randomized to treatment with IMP. Participants must withhold rescue medication for a minimum of 6 hours prior to pulmonary function testing. If the participants has taken rescue medication within 6 hours of planned pulmonary function testing, the visit must be rescheduled. The single-blind run-in inhaler(s) should be collected. The inclusion/exclusion and randomization criteria are evaluated. The participants' diary data are reviewed to determine eligibility. An electrocardiogram (ECG) is recorded and evaluated. The ACT and ACQ-6 are completed and the participants must demonstrate an ACQ-6 score of ≥1.5. A urine pregnancy test is completed for females of childbearing potential. Inhaler training is conducted to ensure proper inhaler use prior to PFT. Participants who meet the criteria are then randomized via the Randomization and Trial Supply Management (RTSM) system only after pre-dose PFT confirms eligibility.

Pulmonary function testing is completed approximately 60 and 30 minutes pre-dose and approximately 5, 15, 30, 45, 60, 120, 180, 240, 300 and 360 minutes after 2 inhalations of the assigned IMP (post-dose).

The 360-minute post-dose assessment should be completed prior to administration of the 2nd daily dosing (mid-day dosing) as part of the QID dosing regimen (as applicable) and this dose may be skipped based on investigator judgement. Additionally, rescue medication should not be given during the serial spirometry testing until after the final timepoint of 360 minutes is complete and the pharmacokinetics sample has been collected unless absolutely necessary based on participant symptoms. Any rescue medication given during the testing must be documented along with the time it was administered (time after dose of IMP).

In a subset of approximately 300 participants, a pre-dose pharmacokinetics sample is obtained followed by a pharmacokinetics sample immediately after the 45-minute spirometry timepoint, and after the 180- and 360-minute spirometry timepoints. The time of IMP dosing and for each pharmacokinetics sample collected is recorded using the clock on the spirometry device as the reference time. The 360-minute post-dose assessment should be completed prior to administration of the second daily dosing (mid-day dosing) as part of the QID dosing regimen (as applicable).

The IMP dosing instructions are reviewed during inhaler training. Participants take 2 inhalations (1 dose) in the early morning, midday, early evening and bedtime as much as possible. Participants should wash their mouth with water without swallowing after using the IMP.

Once all the trial procedures, including re-training on the diary and handheld device used to measure PEF (prior to IMP use) each morning, the participants may be discharged from the clinic.

Double blinded period

The double-blind treatment period will last approximately 4 weeks (V3 through V5). The participants are contacted by telephone to assess adverse events, concomitant medications and review diary and IMP compliance at weeks 1 and 3. At week 3, the participants should be reminded to withhold the morning dose of IMP, and rescue medication for 6 hours (if possible) prior to V5.

Visit 4 (V4) occurs 14±2 days after V3. Vital signs are assessed. The IMP that participants have been using is collected and new IMP dispensed. At this visit, the diary data is reviewed with respect to data entry compliance including morning PEF measurements, compliance with IMP dosing and counselling on the importance of diary compliance to the trial outcome. Participants who demonstrate less than 80% compliance with diary entry and IMP are retrained. All participants must demonstrate proper technique for taking 1 dose (2 inhalations) using the training inhaler. Participants are retrained on inhaler technique as required.

Visit 5 (V5) occurs 14 (±2) days after V4. Participants must withhold the morning daily dose of IMP and rescue medication for a minimum of 6 hours prior to pulmonary function testing. If the participants has taken IMP or used rescue medication within 6 hours of planned pulmonary function testing, the visit must be rescheduled. At this visit, clinical laboratory testing, complete physical examination including vital signs, ECG testing, ACQ-6 and ACT questionnaires are completed. Pulmonary function testing is completed approximately 60 and 30 minutes pre-dose and approximately 5, 15, 30, 45, 60, 120, 180, 240, 300 and 360 minutes after 2 inhalations of assigned IMP (post-dose). Rescue medication should not be given during the serial spirometry testing until after the final timepoint of 360 minutes is complete unless absolutely needed to treat asthma symptoms. Any rescue medication given during the testing (serial spirometry) must be documented along with the time it was administered (time after dose of IMP). After testing is completed, the IMP, daily diary and the handheld PEF device are collected. Diary compliance should be assessed. The participants should re-start their usual asthma maintenance treatment or as advised by the investigator.

Follow-up visit

A follow-up visit (V6) may occur by phone or in the clinic per the investigator's judgement 7 (±3) days after V5 to assess adverse events and concomitant medications.

Planned number of participants and countries

Approximately 1200 individuals are screened to achieve 600 randomized participants 12 years of age and older (150 participants per treatment group).

The trial is conducted in the USA and elsewhere internationally in approximately 100 investigational centers.

Primary and secondary trial objectives and endpoints

The primary and secondary trial objectives and endpoints are set out in Table 1.

Table 1. Primary and secondary trial objectives and endpoints

| Objectives | Endpoints |
|---|---|
| **Primary** | |
| To evaluate the efficacy of Fp/ABS eMDPI | Baseline-adjusted postdose forced expiratory volume in one second ($FEV_1$) area under the effect curve from time zero to 6 hours ($AUEC_{0-6h}$) over 4 weeks ($FEV_1$ $AUEC_{0-6h}$) Baseline-adjusted trough $FEV_1$ at week 4 |
| **Secondary** | |
| To evaluate the efficacy of Fp/ABS eMDPI administered 4 times daily (QID) | Time to 15% improvement from baseline $FEV_1$ postdose on day 1 Time to 12% improvement from baseline $FEV_1$ postdose on day 1 Duration of 15% increase in $FEV_1$ from baseline postdose on day 1 Asthma Control Questionnaire-6 (ACQ-6) response at week 4 defined as achieving a decrease in score from baseline value of at least 0.5 Asthma Control Test (ACT) score response at week 4 defined as achieving an increase in score from baseline value of at least 3 |
| To evaluate the safety and tolerability of Fp/ABS eMDPI administered four times daily over 4 weeks | Occurrence of adverse events during the trial Occurrence of serious adverse events during the trial Trial withdrawal due to treatment-emergent adverse events |
| To investigate the pharmacokinetics of Fp/ABS eMDPI, ABS eMDPI and Fp eMDPI after administration of a single dose | Plasma concentration of Fp and ABS at the specified timepoints |

The primary estimand for the baseline-adjusted postdose $FEV_1$ $AUEC_{0-6h}$ over 4 weeks are set out in Table 2.

Table 2. Primary estimand for the baseline-adjusted postdose $FEV_1$ $AUEC_{0-6h}$ over 4 weeks

| Treatment | Participants are randomly assigned to receive one of the trial treatment regimens shown below in a 1:1:1:1 ratio: Fp/ABS eMDPI 55/117 mcg Fp eMDPI 55 mcg ABS eMDPI 117 mcg Placebo eMDPI |
|---|---|
| Target population | Eligible male and female participants ≥12 years of age who have a GINA-compliant, physician diagnosis of asthma, with a documented history of the last 6 months |
| Primary outcome measure | Baseline-adjusted postdose $FEV_1$ $AUEC_{0-6h}$ over 4 weeks (ABS eMDPI 117 mcg vs placebo eMDPI, Fp/ABS eMDPI 50/117 mcg vs placebo eMDPI, and Fp/ABS eMDPI 55/117 mcg vs Fp eMDPI 55 mcg) |
| Intercurrent events strategy | The following are considered as intercurrent events (ICEs): Occurrence of COVID-19 pneumonia that is clinically diagnosed and requiring systemic therapy or with chronic respiratory sequelae that might change the natural history of the participant's airway disease Any use of systemic corticosteroids (SCS) Discontinuing IMP and switching to an alternative treatment Treatment policy strategy is adopted for the intercurrent events (i.e. whether or not these intercurrent events happen is irrelevant). |

(continued)

| Population-level summary | The population summary is the difference in model adjusted means of baseline-adjusted postdose $FEV_1$ $AUEC_{0-6h}$ over 4 weeks for ABS eMDPI 117 vs placebo eMDPI, Fp/ABS eMDPI 50/117 mcg vs placebo eMDPI, and Fp/ABS eMDPI 55/117 mcg vs Fp eMDPI 55mcg |
|---|---|

The primary estimand for the baseline-adjusted trough $FEV_1$ at week 4 is defined by the attributes set out in Table 3.

Table 3. Primary estimand for the baseline-adjusted trough $FEV_1$ at week 4

| Treatment | Participants are randomly assigned to receive one of the trial treatment regimens shown below in a 1:1:1:1 ratio:<br>Fp/ABS eMDPI 55/117 mcg<br>Fp eMDPI 55 mcg<br>ABS eMDPI 117 mcg<br>Placebo eMDPI |
|---|---|
| Target population | Eligible male and female participants ≥12 years of age who have a GINA-compliant, physician diagnosis of asthma, with a documented history of the last 6 months |
| Primary outcome measure | Baseline-adjusted trough $FEV_1$ at week 4 (Fp eMDPI 55mcg vs placebo eMDPI, Fp/ABS eMDPI 55/117 mcg vs placebo eMDPI, Fp/ABS eMDPI 55/117 mcg vs ABS eMDPI 117 mcg) |
| Intercurrent events strategy | The following are considered as intercurrent events (ICEs):<br>Occurrence of COVID-19 pneumonia that is clinically diagnosed and requiring systemic therapy or with chronic respiratory sequelae that might change the natural history of the participant's airway disease<br>Any use of SCS<br>Discontinuing IMP and switching to an alternative treatment<br>Treatment policy strategy is adopted for the intercurrent events (i.e. whether or not these intercurrent events happen is irrelevant). |
| Population-level summary | The population summary is the difference in model adjusted means of baseline-adjusted trough $FEV_1$ at week 4 for Fp eMDPI 55mcg vs placebo eMDPI, Fp/ABS eMDPI 55/117 mcg vs placebo eMDPI, Fp/ABS eMDPI 55/117 mcg vs ABS eMDPI 117 mcg |

The rationale for the primary estimands is to precisely define the treatment effect of Fp/ABS eMDPI. The ICEs (as defined above) after the first treatment dose will have an impact on the measurements of the co-primary endpoints (i.e. baseline-adjusted postdose $FEV_1$ $AUEC_{0-6hr}$ over 4 weeks; and baseline-adjusted trough $FEV_1$ at week 4), as they are expected to be events that will occur in clinical trials, as well as in clinical practice. The treatment policy defined in the primary estimates demonstrates the intent-to-treat (ITT) principle described in the ICH E9, i.e., the data will be analyzed regardless of whether the ICEs occur, thus will provide reliable estimation of treatment effect.

Exploratory objectives and endpoints

The exploratory trial objectives and endpoints are set out in Table 4.

Table 4. Exploratory trial objectives and endpoints

| Objectives | Endpoints |
|---|---|
| **Exploratory** | |
| To characterize the safety of Fp/ABS 55/117 mcg eMDPI compared to mono-components and to placebo over the 4 week treatment period | Vital signs, physical examination (including oropharyngeal), clinical laboratory tests and electrocardiogram (ECG) findings<br>Concomitant medication use throughout the trial |

(continued)

| Objectives | Endpoints |
|---|---|
| **Exploratory** | |
| To characterize the lung function measurements of Fp/ABS 55/117 mcg eMDPI compared to mono-components and to placebo over the 4 week treatment period | Change from baseline in the weekly average of the daily morning trough (pre-dose) $FEV_1$ measured daily by handheld device over weeks 1-4. Note: baseline is defined as the average daily morning trough (pre-dose) $FEV_1$ over the 7 days prior to visit 3 (day of randomization) |
| To characterize the symptom scores of Fp/ABS 55/117 mcg eMDPI compared to mono-components and to placebo over the 4 week treatment period | Change from baseline in the weekly average nighttime asthma symptom score over weeks 1-4. Note: baseline score is defined as the average score of the 7 days prior to visit 3 (day of randomization)<br><br>Change from baseline in the weekly average daytime asthma symptom score over weeks 1-4. Note: baseline score is defined as the average score of the 7 days prior to visit 3 (day of randomization) |
| To characterize the asthma control of Fp/ABS 55/117 mcg eMDPI compared to mono-components and to placebo over the 4 week treatment period | Change from baseline in the weekly average of total daily (24-hour) use of rescue albuterol (salbutamol)<br><br>Time to first severe clinical asthma exacerbation (sCAE) |

Trial population

This trial will enroll participants with mild to moderate asthma in accordance to GINA 2023 guidelines. The participants to be studied in this trial include patients with mild to moderate asthma who have demonstrated at least 12% response to bronchodilator testing during the screening period. The participants have also demonstrated the required lung function and have also demonstrated compliance with study procedures and are uncontrolled based on the ACQ-6. This population is selected to be consistent with asthma patients who are anticipated to be treated with the combination product as rescue therapy.

Inclusion criteria

Participants may be included in this trial only if they meet all of the following criteria.

(a) The participant is capable of giving signed informed consent (≥18 years of age). Participants 12 to 17 years of age (or as applicable per local requirements) are able to provide assent and written consent must be provided by a parent/legally acceptable representative before any trial procedures are performed.
(b) The participant is a female or male 12 years of age or older at the time of informed consent or as allowed by local regulation.
(c) The participant has a diagnosis of asthma in accordance with GINA 2023 guidelines or NHLBI 2020 guidelines (National Heart, Lung, and Blood Institute of the National Institutes of Health) of at least 6 months duration.
(d) The participant has an ACQ-6 score of ≥1.5 at the Baseline Visit 3.
(e) Lung Function: A participant has a pre-bronchodilator $FEV_1$ of ≥50 to 85% predicted normal for adults (≥18 years of age) and $FEV_1$ of ≥50 to 90% predicted normal for participants 12 to 17 years of age demonstrated during the screening period (P.H. Quanjer et al., Eur Respir J, 2012, 40(6), 1324-1343).
(f) The participant demonstrates age-appropriate spirometry performance during the screening visit (V1) (i.e. meets the American Thoracic Society/European Respiratory Society acceptability/repeatability criteria [B.L. Graham et al., Standardization of Spirometry 2019 Update, An Official American Thoracic Society and European Respiratory Society Technical Statement, Am J Respir Crit Care Med, 2019; 200(8), 15]).
(g) Response to bronchodilator testing (Reversibility): Participants must demonstrate at least 12% change from baseline in $FEV_1$ after treatment with 2 to 4 inhalations of albuterol (salbutamol) HFA 90 mcg per inhalation. Participants aged ≥18 years must also demonstrate at least 200 ml increase from baseline $FEV_1$.
(h) The participant demonstrates proper technique using the training inhaler at V2 (beginning of the run-in period).
(i) The participant is capable of performing PEF measurements using the handheld device as judged by the investigator at V2.

(j) The participant must be receiving 1 of the following medications to treat asthma (as maintenance) with no changes for 30 days prior to V1:

(i) NCS therapy including SABA alone or in combination with leukotriene modifiers or theophylline and
(ii) low, medium or high dose ICS therapy with or without a LABA and/or in combination with NCS therapy as above.

(k) If female, a participant is currently not pregnant, breastfeeding, or attempting to become pregnant (for at least 30 days before the screening visit and throughout the duration of the trial), or is of non-childbearing potential, defined as any of the following:

(i) premenarche
(ii) at least 1 year postmenopausal (no menses for at least 12 months without an alternative medical cause plus an increased concentration of FSH of more than 35 U/L in women not using hormonal contraception or hormonal replacement therapy)
(iii) surgically sterile (bilateral tubal ligation, bilateral oophorectomy, salpingectomy, or hysterectomy)
(iv) congenitally sterile (as assessed by a physician)
(v) diagnosed as infertile and not undergoing treatment to reverse infertility OR, if of childbearing potential, has a negative serum β-human chorionic gonadotropin (β-HCG) test at screening visit and is willing to commit to using a medically accepted, highly effective method of birth control as defined below for the duration of the trial:
(vi) combined estrogen and progestogen hormonal contraception (oral, intravaginal, transdermal) associated with inhibition of ovulation; these should be initiated at least 30 days before the first dose of IMP and maintained throughout the trial.
(vii) progestogen-only hormonal contraception (oral, injectable, implantable) associated with inhibition of ovulation; these should be initiated at least 30 days before the first dose of IMP and maintained throughout the trial.
(viii) intrauterine device and intrauterine hormone-releasing system need to be in place at least 2 months before screening and maintained throughout the trial.
(ix) bilateral tubal occlusion, except for hysteroscopic bi-tubal ligation for which a hysterosalpingogram is required 3 months post procedure to assess surgical success
(x) vasectomized partner provided he is the sole sexual partner and has received medical assessment of the surgical success
(xi) sexual abstinence - refraining from heterosexual intercourse during the trial period Of note, if a female participant has reached puberty and achieved menarche (as determined by the investigator), the participant must be counselled regarding the possible unknown risks associated with IMP during pregnancy (following permission of the parent/legally acceptable representative).

(l) The participant must be willing and able to comply with trial restrictions and to remain at the investigational center for the required duration during the trial period, and willing to return to the investigational center for further visits, as applicable, and the follow up procedures and assessments as specified in this protocol.

Exclusion criteria

Participants are excluded from participating in this trial if they meet any of the following criteria.

(a) The participant has a history of life-threatening asthma defined as any history of significant asthma episode(s) requiring intubation, associated with hypercapnia, respiratory arrest, hypoxic seizures or an asthma related syncopal episode.
(b) The participant has experienced an asthma exacerbation requiring treatment with systemic corticosteroids within 12 months prior to the screening visit.
(c) The participant has any medical or psychiatric condition that, in the opinion of the investigator, could jeopardize or would compromise the Participant's ability to participate in this trial or compromise the interpretation of the trial measures and endpoints.
(d) The participant is currently being treated with a prohibited medication or has been treated with a prohibited medication within the washout periods.
(e) The participant has cancer not in complete remission for at least 5 years. Note: Participants with fully resected basal cell or squamous cell carcinoma of the skin, localized prostate cancer or carcinoma in situ of the cervix may be included if the investigator believes the condition has been clinically controlled and would not represent a safety concern.

(f) The participant has previously participated in this trial or previously participated in a trial with albuterol sulfate) as IMP.

(g) The participant has had an upper or lower respiratory tract infection within 2 weeks or has had a confirmed case of COVID-19 within 6 weeks prior to V1. Symptoms of the infection(s) must be completely resolved prior to entering screening.

(h) The participant has participated as a randomized Participant in any investigational drug trial within 30 days or within 5 half-lives of the IMP under study (if treated) preceding screening or plans to participate in another investigational drug or device trial at any time during this trial. Note: Prior depemokimab exposure is prohibited without exception.

(i) The participant is a current tobacco smoker or has a smoking history of ≥10 pack-years, or the participant used tobacco within the past 6 months (e.g. cigarettes, cigars, chewing tobacco, or pipe tobacco), or the participant has a history of "vaping" tobacco, marijuana, or any other substance within 24 months. Note: Participants with a positive urinary cotinine test are excluded.

(j) The participant has known hypersensitivity to any corticosteroid, albuterol (salbutamol), or any of the excipients (i.e. lactose) in the IMP formulations. Note: Dietary lactose intolerance does not exclude the participant from participation in the trial or as per the investigator's medical decision.

(k) The participant has another confounding underlying lung disorder (e.g. chronic obstructive pulmonary disease, chronic bronchitis, emphysema, bronchiectasis with the need of treatment, cystic fibrosis, pulmonary fibrosis).

(l) The participant has a clinically significant abnormal ECG finding at baseline.

(m) The participant is an employee or relative of an employee who works at any clinical research center taking part in this trial.

(n) More than 1 participant from the same household are excluded unless the first participant completes the trial and has returned IMP. Then, the second household member may be screened and included, as applicable.

(o) Vulnerable participants (e.g. people kept in detention).

Prohibited medications are set out in Table 5.

Table 5. Prohibited medications

| Type of Medication | Washout period before the screening visit (unless otherwise specified) |
|---|---|
| Biologic medications used to treat asthma (e.g. Omalizumab, benralizumab, mepolizumab, reslizumab, dupilumab, tezepelumab) | 5 half-lives |
| Depemokimab (GSK3511294) | Any exposure prohibited |
| Monoclonal antibodies used to treat malignancy | Any exposure prohibited |
| Any other investigational drug | 5 half-lives |
| Corticosteroids (oral, iv, intra-articular, intramuscular) | 30 days |
| Immunologically active biologic medications (e.g. antitumor necrosis factor alpha, abatacept) | 90 days |
| Immunosuppressive therapy (e.g. methotrexate, gold, azathioprine) | 90 days |
| Immunotherapy[a] | Initiation within 90 days or change in dose within 30 days |
| Strong Cytochrome P3A4 inhibitors (e.g. ritonavir, ketoconazole, itraconazole, clarithromycin, cobicistat, etc) | 14 days |
| Inhaled cromolyn preparations | 14 days |
| Inhaled short-acting muscarinic antagonists (SAMA) or long-acting muscarinic antagonists (LAMA) | 7 days |
| Phosphodiesterase-4 inhibitors (e.g. roflumilast)[b] | 5 half-lives |
| Monoamine oxidase inhibitors (e.g. phenelzine, selegiline) | 14 days |
| Tricyclic anti-depressants (e.g. amitriptyline, doxepin, desimparamine) | 14 days |

(continued)

| Type of Medication | Washout period before the screening visit (unless otherwise specified) |
|---|---|
| Non-selective $\beta_2$ adrenergic blockers including eye medications[c] | 14 days |
| Inhaled corticosteroids (other than IMP) | Discontinue at V2 |
| Long acting $\beta_2$ agonists (LABA) | Discontinue at V2 |
| Theophylline | Discontinue at V2 |
| Leukotriene modifiers (e.g. montelukast, zileuton) | Discontinue at V2 |
| Any medications including inhaled, topical, or nasal preparations containing fluticasone propionate or fluticasone furoate are prohibit from V2 until trial procedures have been completed at V5[d]. | Discontinue at V2 |

a Immunotherapy for the treatment of allergies by any route is permitted as long as therapy was initiated 90 days or more before screening and the participant has been on a stable dose for 30 days or more before screening. The participant must remain on this stable regimen throughout the trial.
b PDE-5 inhibitors (e.g. sildenafil, tadalafil) are not prohibited.
c Cardio-selective ($\beta$1) adrenergic blockers with a low daily dose are allowed (e.g. metoprolol $\leq$100mg per day).
d A non-fluticasone nasal corticosteroid may be substituted based on the investigator's discretion. iv=intravenous; LABA=long-acting $\beta$2 agonist; CYP=cytochrome P450.

Randomization criteria

Participants who meet all of the randomization criteria and none of the exclusion criteria may be randomized into this trial:

(a) the participant remains in general good health and continues to qualify based on all of the inclusion and exclusion criteria;
(b) the participant has not required any treatment for asthma other than single-blind run-in medication and trial supplied SABA (rescue medication) during the run-in period;
(c) the participant has an ACQ-6 score of $\geq$1.5 at V3;
(d) the participant has not had an upper- or lower- respiratory infection during the run-in period;
(e) the participant has not used any of the prohibited medications during the run-in period; and
(f) the participant has complied with home spirometry and diary entry on at least 5 of the last 7 days prior to Visit 3 including:

(i) completion of daytime and nighttime asthma symptom scores;
(ii) recording of daytime and nighttime rescue medication (SABA) use (whether used or not);
(ii) completion of the morning PEF assessments by handheld spirometer;
(iv) recording of the single-blind inhaler use; and

(g) an average of the 60 and 30 minute pre-dose FEV$_1$ assessments is between $\geq$50 to 85% predicted normal for adults ($\geq$18 years of age) and FEV$_1$ of $\geq$50 to 90% predicted normal for participants 12 to 17 years of age.

Individuals who do not meet criteria for trial eligibility must not be enrolled via protocol waivers or exemptions.

Trial intervention and concomitant therapy

The IMPs used in this trial are defined as the test IMP, active control IMPs, and placebo.
The test IMP is Fp/ABS eMDPI inhalation powder, which is further described in Table 6.
Participants receive the test IMP at dose of 55/117 mcg, with a dosing frequency of 2 inhalations QID. The maximum daily dose is expected to be 8 inhalations (4 doses) per day.
Participants are advised to rinse their mouth with water without swallowing, after inhalation.

Reference investigational medicinal products

The active control reference IMPs are ABS eMDPI inhalation powder and Fp eMDPI inhalation powder, which are further described in Table 6.

Participants will receive the active control IMP at a dose of 117 mcg for ABS, or 55 mcg for Fp, with a dosing frequency of 2 inhalations QID. Participants should separate the doses beginning in the early morning, mid-day, early evening and at bedtime as much as possible. The maximum daily dose is expected to be 8 inhalations (4 doses) per day.

Participants will be advised to rinse their mouth with water without swallowing, after inhalation.

Additional details for ABS may be found in the United States Prescribing Information (USPI) for ProAir® Digihaler®, June 2022 and ProAir® RespiClick®, September 2020. Additional details for Fp may be found in the USPI for ArmonAir® Digihaler®, March 2021, and ArmonAir® RespiClick®, July 2021 Participants will receive the active control IMP at a dose of 117 mcg for ABS, or 55 mg for Fp, with a dosing frequency of 2 inhalations QID. The maximum daily dose is expected to be 8 inhalations (4 doses) per day.

Placebo investigational medicinal products

Patients will receive the placebo as 2 inhalations QID (Table 6). Participants should separate the doses beginning in the early morning, mid-day, early evening and at bedtime as much as possible and rinse their mouths with water without swallowing after each dose.

The inhaler device

The inhaler device manufactured by Teva provided for use in this trial is the eMDPI (Digihaler®) device, which contains a built-in eModule that detects, records, and stores data on inhaler events.

In the current trial, the eModule component will not be used in conjunction with a mobile application. The inhalers are returned to a central site and the data are downloaded in compliance with all applicable Data protection policies apply.

All participants are provided training on use of a training inhaler device during V2 and V3. Participants must be able to use the device without a spacer device due to incompatibility. Spacers are not allowed for use with the eMDPI device. The training is done after the screening visit when the participant has been deemed eligible for inclusion and is commencing the run-in period. Directions for use of the device are reviewed with each participant. Participants will then be provided with an inhaler training device. The training devices are empty and do not contain active medication. Participants will practice using the device and are required to demonstrate their understanding of the correct technique. To ensure the correct technique for the device, it is used over the duration of the trial, training procedures should be repeated and documented at each clinic visit for participants who demonstrate incorrect technique during the evaluation of inhaler use. Sites are responsible for labeling and storing the training inhaler devices between visits.

The IMPs and device used in this trial are described in Table 6.

Table 6. Investigational medicinal products and device used in the trial

| IMP type | Test IMP | Active Control IMP | Active Control IMP | Placebo IMP |
|---|---|---|---|---|
| **IMP name** | Fp/ABS eMDPI | Fp eMDPI | ABS eMDPI | Placebo eMDPI |
| **Formulation** | Inhalation powder | Inhalation powder | Inhalation powder | Inhalation powder |
| **Unit metered dose strength(s)/Dosage level(s)** | 55/117 mcg | 55 mcg | 117 mcg | 0 mcg |
| **Route of administration** | Inhalation | Inhalation | Inhalation | Inhalation |
| **Dosing instructions** | 2 inhalations QID | 2 inhalations QID | 2 inhalations QID | 2 inhalations QID |
| **Intervention type** | Combination product | Drug | Drug | Placebo |
| **Packaging** | Inhaler device | Inhaler device | Inhaler device | Inhaler device |
| **Device type** | Standard flow | Standard flow | Standard flow | Standard flow |
| ABS=albuterol sulfate; eMDPI=multidose dry powder inhaler with integrated electronic module; Fp=fluticasone propionate; IMP=investigational medicinal product; QID=4 times a day. | | | | |

Non-investiqational medicinal products

Rescue therapy

During the run-in and treatment periods of the trial, participants are supplied with albuterol (salbutamol) sulfate HFA pMDI inhalation aerosol 90 mcg ex-actuator as rescue medication by the investigational center. Albuterol sulfate HFA pMDI is a SABA to be used as needed to control symptoms of asthma.

Dosing is 2 inhalations every 4 to 6 hours as needed to control asthma symptoms. Participants should not exceed more than 12 inhalations per day.

Although the use of the rescue medication is allowable at any time during the trial, participants must withhold rescue medication for a minimum of 6 hours prior to any pulmonary function testing.

The date and time of rescue medication administration is recorded.

Efficacy assessments and procedures

Pulmonary function tests

Formal clinic-based pulmonary function testing (spirometry and reversibility testing) is conducted in the morning between 0530 and 1100 during the screening period. Participants are required to withhold asthma maintenance medications for at least approximately 24 hours prior to lung function testing for once daily medications or 12 hours for twice daily, or more frequently dosed, medication. If the participant has taken asthma maintenance medication within 24 $\pm 2$ (daily dosed) or 12$\pm 2$ hours (dosed twice daily or more frequently) or SABA within 6 hours of the planned pulmonary function testing or ICS/LABA used as rescue medication within 12 hours of the planned pulmonary function testing, the visit must be rescheduled.

Airway reversibility (response to bronchodilator testing), as measured by forced expiratory volume in 1 second ($FEV_1$), at the screening visit (V1) is demonstrated after pre-albuterol pulmonary function testing is completed via central spirometry and then repeating $FEV_1$ measurement approximately 15-45 minutes after receiving 2-4 inhalations (based on investigator judgment) SABA medication. If a participants $FEV_1$ improves at least 12% between the 2 tests and an increase of 200 mL (for participants $\geq$18 years of age only) the participants are deemed to have met the response to bronchodilator requirement. Participants 12-17 years of age do not need to meet the 200 mL requirement. Historical reversibility is not acceptable.

Baseline spirometry is conducted in the morning between approximately 0530 to 1100 hours at visit 3. Subsequent testing (V5) should begin within approximately 1 hour of the baseline timepoint. Participant must have withheld rescue medication (SABA) and IMP for at least 6 hours prior to any spirometry testing or the visit must be rescheduled.

Baseline spirometry measurements for $FEV_1$ and FVC are defined as the average of the measurements obtained at the predose timepoints at V3. If only one measure is available, then that single measurement is defined as baseline.

Baseline predose spirometry occurs at the timepoints listed. Participants are allowed up to 8 attempts (for each timepoint) in order to have 2 technically acceptable and repeatable measures (B.L. Graham et al., Standardization of Spirometry 2019 Update. An Official American Thoracic Society and European Respiratory Society Technical Statement. Am J Respir Crit Care Med., 2019, 200(8), 15).

Participants are allowed up to 5 attempts for each postdose timepoint and may stop after 2 technically acceptable measures have been completed. The highest $FEV_1$ from the 2 acceptable curves are used as the result for that timepoint. If only one acceptable measure is obtained, it is used and if no acceptable measures are obtained, the best spirogram is used for the timepoint.

Once the baseline measurement is available and meets the other trial requirements for inclusion, the participant may be randomized (as applicable).

Participants are not randomized until the predose testing is completed and confirmed to meet the trial requirements. The IMP should be dispensed and the participants may be dosed (after a predose PK sample has been collected) with IMP. The dose is 2 inhalations. Dosing should be completed with the second dose occurring approximately 30 seconds after the first. The time of dosing is based on the first inhalation.

Serial spirometry assessments and permitted time windows are detailed below in Table 7.

Table 7. Serial Spirometry Assessment Schedule

| Time | Permitted time window |
|---|---|
| 60-minute predose | $\pm$15 minutes |
| 30-minute predose | $\pm$15 minutes |

(continued)

| Time | Permitted time window |
|---|---|
| 5-minute postdose | ±2 minutes |
| 15-minutes postdose | ±3 minutes |
| 30-minutes postdose | ±3 minutes |
| 45-minutes postdose | ±3 minutes |
| 60-minutes postdose | ±5 minutes |
| 120-minutes postdose | ±10 minutes |
| 180-minutes postdose | ±10 minutes |
| 240-minutes postdose | ±10 minutes |
| 300-minutes postdose | ±10 minutes |
| 360-minutes postdose | ±10 minutes |

Asthma control questionnaire 6-item version

The ACQ-6 is a shortened version of the validated asthma assessment tool (i.e. ACQ) that has been widely used (E.F. Juniper et al., Development and validation of a questionnaire to measure asthma control, Eur Respir J., 1999, 14(4), 902-907) and includes 6 questions that are self-assessments (completed by the participant). Each item on the ACQ-6 has a possible score ranging from 0 to 6, and the total score is the mean of all responses.

Asthma control test

The Asthma Control Test (ACT) is a simple, participant-completed tool used for the assessment of overall asthma control in participants 12 years of age and older. The 5 items included in the ACT assess daytime and nighttime asthma symptoms, use of rescue medication, and impact of asthma on daily functioning. Each item in the ACT is scored on a 5-point scale, with summation of all items providing scores ranging from 5 to 25. The scores span the continuum of poor control of asthma (score of 5) to complete control of asthma (score of 25), with a cutoff score of 19 and below indicating participants with poorly controlled asthma (R.A. Nathan et al., Development of the asthma control test: a survey for assessing asthma control, J Allergy Clin Immunol, 2004, 113(1), 59-65; M. Schatz et al., Asthma Control Test: reliability, validity, and responsiveness in participants not previously followed by asthma specialists, J Allergy Clin Immunol, 2006,117(3), 549-56).

Peak expiratory flow

Daily morning PEF measurements are performed by the participant using a portable handheld device. Results are recorded in the participant diary. Participants are trained on proper PEF measurements at V2.

Asthma symptom score

The asthma symptom score is determined from the information recorded in the participant Diary. A Likert style scale is used to quantify symptomatology. This scale has been used previously to assess changes in asthma symptoms in response to novel asthma treatments (G.S. Shapiro et al., Comparable bronchodilation with hydrofluoroalkane 134a (HFA) albuterol and chlorofluorocarbons 11/12 (CFC) albuterol in children with asthma, J Asthma, 2000, 37(8), 667 75).
Asthma symptom scores should be determined twice daily, in the morning and evening.
Daytime Symptom Score (determined in the evening):

0=No symptoms during the day
1=Symptoms for 1 short period during the day
2=Symptoms for 2 or more short periods during the day
3=Symptoms for most of the day which did not affect my normal daily activities
4=Symptoms for most of the day which did affect my normal daily activities
5=Symptoms so severe that I could not go to work or perform normal daily activities

Nighttime Symptom Score (determined in the morning):

0=No symptoms during the night
1=Symptoms causing me to wake once (or wake early)
2=Symptoms causing me to wake twice or more (including waking early)
3=Symptoms causing me to be awake for most of the night
4=Symptoms so severe that I did not sleep at all

For any given day, the nighttime score recorded in the morning and the daytime score recorded in the evening of the same day will serve as 1 day for total score and rescue medication (i.e. IMP) use (24 hour periods).

Symptom free days are defined as 24 hour periods with asthma symptom score of 0.

Asthma control days are defined as 24 hour periods with asthma symptom scores of 0 and no rescue medication use.

Serious clinical asthma exacerbation

A sCAE is defined as a worsening (deterioration) of asthma resulting in at least one of the following:

- treatment with SCS for at least 3 consecutive days to treat symptoms of asthma worsening. A single depo-injection of corticosteroids is considered equivalent to a 3-day course of oral corticosteroids
- an urgent care or emergency room visit due to asthma that required SCS for at least 3 consecutive days
- a hospitalization (defined as admission to an in-participant facility or observation unit) for ≥24 hours due to asthma

All severe CAEs that occur after V3 must be identified and recorded in the CRF including the details of the treatment. Severe CAEs that occur ≥7 days after treatment is completed for a severe CAE are considered separate exacerbations. Severe CAEs that occur <7 days after treatment are considered the same episode. The first day of a severe CAE is the day that treatment was initiated, and the last day is the day, treatment is completed. For participants who receive depo-injection of a corticosteroid, the treatment completion is 3 days including the day of administration.

All protocol defined severe CAE must also have at least one sign or symptom criteria from the following: chest tightness; cough; shortness of breath; change in sputum; nighttime awakening due to asthma; limitation of activity due to asthma; decreased $FEV_1$; or decreased PEF.

Investigator defined exacerbation: If an investigator determines that a participant is experiencing a severe CAE but does not meet the symptom requirements per protocol, they must justify the decision and document the reason(s) in the CRF.

The recommended treatment (Global Initiative for Asthma (GINA) Report, updated 2022, Global Strategy for Asthma Management and Prevention, available at: www.ginasthma.org) for a severe exacerbation is prednisolone 1 mg/kg/day up to 50 mg for adults and adolescents, once per day (preferably in the morning) for 5 to 7 days.

**Claims**

1. A method of treatment of asthma comprising a pro re nata (PRN) administration of a fixed-dose dry powder inhalation composition comprising fluticasone propionate and albuterol sulfate as a rescue medication in patients, wherein the fluticasone propionate is administered at a delivered dose of 22-59 mcg per inhalation and the albuterol sulfate is administered at a delivered dose of 92-124 mcg per inhalation, and wherein the treatment provides an improved baseline-adjusted postdose forced expiratory volume in one second ($FEV_1$) area under the effect curve from time zero to 6 hours ($AUEC_{0-6h}$) when compared to fluticasone propionate treatment and an improved baseline-adjusted trough $FEV_1$ when compared to albuterol sulfate treatment.

2. The method of claim 1, wherein the asthma treatment comprises one or more of: a reduced time to 15% improvement from baseline $FEV_1$ postdose when compared to placebo, fluticasone propionate or albuterol sulfate treatment; a reduced time to 12% improvement from baseline $FEV_1$ postdose when compared to placebo, fluticasone propionate or albuterol sulfate treatment; an increased duration of 15% increase in $FEV_1$ from baseline postdose when compared to placebo, fluticasone propionate or albuterol sulfate treatment; an improved Asthma Control Questionnaire-6 (ACQ-6) response defined as achieving a decrease in score from baseline value of at least 0.5 when compared to placebo, fluticasone propionate or albuterol sulfate treatment; and/or an improved Asthma Control Test (ACT) score response defined as achieving an increase in score from baseline value of at least 3 when compared to placebo, fluticasone propionate or albuterol sulfate treatment.

3. The method of claim 1, wherein the fluticasone propionate is administered at a delivered dose of 23-56 mcg per inhalation and the albuterol sulfate is administered at a delivered dose of 97-119 mcg per inhalation.

4. The method of claim 3, wherein the fluticasone propionate is administered at a delivered dose of 25-54 mcg per inhalation and the albuterol sulfate is administered at a delivered dose of 103-113 mcg per inhalation.

5. The method of claim 4, wherein the fluticasone propionate is administered at a delivered dose of 26 or 51 mcg per inhalation and the albuterol sulfate is administered at a delivered dose of 108 mcg per inhalation.

6. The method of claim 1, wherein the fluticasone propionate is administered at a metered dose of 26-63 mcg per inhalation and the albuterol sulfate is administered at a metered dose of 99-135 mcg per inhalation.

7. The method of claim 6, wherein the fluticasone propionate is administered at a metered dose of 27-61 mcg per inhalation and the albuterol sulfate is administered at a metered dose of 105-129 mcg per inhalation.

8. The method of claim 7, wherein the fluticasone propionate is administered at a metered dose of 29-58 mcg per inhalation and the albuterol sulfate is administered at a metered dose of 111-123 mcg per inhalation.

9. The method of claim 8, wherein the fluticasone propionate is administered at a metered dose of 30 or 55 mcg per inhalation and the albuterol sulfate is administered at a metered dose of 117 mcg per inhalation.

10. The method of claim 1, wherein the fixed dose combination of fluticasone propionate and albuterol sulfate is administered at a maximum daily dose that does not exceed 12 inhalations (e.g. 2 inhalations 6 times a day).

11. The method of claim 1, wherein the patients are separately administered a maintenance dose of a long-term asthma medication.

12. The method of claim 11, wherein the long-term asthma medication is an ICS, LABA, LAMA, SAMA, biological medication or combinations thereof.

13. The method of claim 12, wherein the ICS is selected from flunisolide, fluticasone propionate, fluticasone furoate, beclomethasone dipropionate, budesonide, mometasone furoate, ciclesonide and combinations thereof.

14. The method of claim 12, wherein the LABA is selected from formoterol, arformoterol, salmeterol, bambuterol, clenbuterol, abediterol, indacaterol, olodaterol, vilanterol, carmoterol and combinations thereof.

15. The method of claim 12, wherein the LAMA is selected from tiotropium, glycopyrronium, umeclidinium, aclidinium, darotropium and combinations thereof.

16. The method of claim 12, wherein the SAMA is ipratropium.

17. The method of claim 12, wherein the biological medication is selected from tezepelumab, dupilumab, mepolizumab, reslizumab, benralizumab, omalizumab, palivizumab and combinations thereof.

18. The method of claim 11 wherein the maintenance therapy daily metered dose is low-, medium- or high-dose depending on the type of medicament and patient's age.

19. The method of claim 18, wherein for patients aged 12 years and older the ICS daily metered doses are: beclomethasone dipropionate, low-dose 200-500 mcg, medium-dose >500-1,000 mcg, high-dose >1,000 mcg; beclomethasone dipropionate ultra-fine particle, low-dose 100-200 mcg, medium-dose >200-400 mcg, high-dose >400 mcg; budesonide, low-dose 200-400 mcg, medium-dose >400-800 mcg, high-dose >800 mcg; ciclesonide, low-dose 80-160 mcg, medium-dose >160-320 mcg, high-dose >320 mcg; fluticasone furoate, low-dose and medium-dose 100 mcg, high-dose 200 mcg; fluticasone propionate, low-dose 100-250 mcg, medium-dose >250-500 mcg, high-dose >500 mcg; and mometasone furoate, low-dose and medium-dose 200-400 mcg, high-dose is >400 mcg.

20. The method of claim 18, wherein for patients aged 6 to <12 years old the ICS daily metered doses are: beclomethasone dipropionate, low-dose 100-200 mcg, medium-dose >200-400 mcg, high-dose >400 mcg; beclomethasone dipropionate ultra-fine particle, low-dose 50-100 mcg, medium-dose >100-200 mcg, high-dose >200 mcg; budesonide, low-dose 100-200 mcg, medium-dose >200-400 mcg, high-dose is >400 mcg; budesonide nebules, low-dose 250-500 mcg, medium-dose >500-1,000 mcg, high-dose >1,000 mcg; ciclesonide, low-dose 80 mcg, medium-dose >80-160 mcg, high-dose >160 mcg; fluticasone furoate, low-dose and medium-dose 50 mcg; fluticasone propionate, low-dose 50-100 mcg, medium-dose >100-200 mcg, high-dose >200 mcg; and mometasone furoate, low-dose and medium-dose 100 mcg, high-dose 200 mcg.

21. The method of claim 1, wherein the patients are any one of GINA step 1-5 patients.

22. The method of claim 1, wherein fixed-dose dry powder inhalation composition consists of fluticasone propionate, albuterol sulfate and alpha-lactose monohydrate.

FIG. 1

**FIG. 2**

102

128

120

129

140

146

146

104

108

FIG. 3

102

148

146

142

140

145

144

122

130

124

126

120

**FIG. 4**

FIG. 5

FIG. 6

FIG. 7

**FIG. 8**

**FIG. 9**

**FIG. 10**

Europäisches
Patentamt
European
Patent Office
Office européen
des brevets

# EUROPEAN SEARCH REPORT

**Application Number**

EP 24 18 9576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/125882 A1 (MATZ JONATHAN [US]) 23 May 2013 (2013-05-23) | 1-7,10, 21 | INV. A61K31/137 |
| Y | * claim 1 * * paragraphs [0008], [0018], [0020], [0033], [0045] * | 1-22 | A61K9/00 A61K31/56 A61K31/58 A61M15/00 |
| Y | US 2024/033222 A1 (O'NEILL BRIAN PAUL [IE] ET AL) 1 February 2024 (2024-02-01) * claims 1, 6 * * paragraphs [0024], [0054], [0124], [0125], [0126], [0132], [0133] * * figures 1-22 * | 1-22 | A61P11/06 |
| Y | DATABASE EMBASE [Online] STN; 14 April 2016 (2016-04-14), Setyoningrum R A: "Comparative Efficacy of Combination Nebulized Salbutamol and Fluticasone Propionate and Nebulized Salbutamol in Children with Mild Moderate Asthma Attack", XP093169189, Database accession no. EMB-002002901066 * abstract * | 1-22 | |
| Y | US 2023/302238 A1 (MILTON-EDWARDS MARK [GB] ET AL) 28 September 2023 (2023-09-28) * paragraphs [0050], [0052], [0053] * | 1-22 | |

**TECHNICAL FIELDS SEARCHED (IPC)**

A61M
A61K
A61P

The present search report has been drawn up for all claims

| Place of search | Date of completion of the search | Examiner |
|---|---|---|
| Munich | 16 December 2024 | Olausson Boulois, J |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons
.............................................................................
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 18 9576

This annex lists the patent family members relating to the patent documents cited in the above-mentioned European search report.
The members are as contained in the European Patent Office EDP file on
The European Patent Office is in no way liable for these particulars which are merely given for the purpose of information.

16-12-2024

| Patent document cited in search report | Publication date | Patent family member(s) | | Publication date |
|---|---|---|---|---|
| US 2013125882 A1 | 23-05-2013 | US | 2013125882 A1 | 23-05-2013 |
| | | WO | 2013075086 A1 | 23-05-2013 |
| US 2024033222 A1 | 01-02-2024 | AU | 2021324395 A1 | 16-03-2023 |
| | | CA | 3189493 A1 | 17-02-2022 |
| | | CN | 116615201 A | 18-08-2023 |
| | | EP | 4196105 A1 | 21-06-2023 |
| | | IL | 300652 A | 01-04-2023 |
| | | JP | 2023539073 A | 13-09-2023 |
| | | KR | 20230066364 A | 15-05-2023 |
| | | PE | 20231846 A1 | 21-11-2023 |
| | | US | 2022047610 A1 | 17-02-2022 |
| | | US | 2023255983 A1 | 17-08-2023 |
| | | US | 2024033222 A1 | 01-02-2024 |
| US 2023302238 A1 | 28-09-2023 | AU | 2021328720 A1 | 02-03-2023 |
| | | CA | 3190456 A1 | 24-02-2022 |
| | | CN | 115942966 A | 07-04-2023 |
| | | EP | 4200871 A1 | 28-06-2023 |
| | | JP | 2023540891 A | 27-09-2023 |
| | | KR | 20230054689 A | 25-04-2023 |
| | | US | 2023302238 A1 | 28-09-2023 |
| | | WO | 2022038275 A1 | 24-02-2022 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016090260 A
- WO 2020222146 A

- US 2015099726 A1

**Non-patent literature cited in the description**

- **P.H. QUANJER et al.** *Eur Respir J*, 2012, vol. 40 (6), 1324-1343
- **B.L. GRAHAM et al.** Standardization of Spirometry 2019 Update, An Official American Thoracic Society and European Respiratory Society Technical Statement. *Am J Respir Crit Care Med*, 2019, vol. 200 (8), 15
- **B.L. GRAHAM et al.** Standardization of Spirometry 2019 Update. An Official American Thoracic Society and European Respiratory Society Technical Statement.. *Am J Respir Crit Care Med.*, 2019, vol. 200 (8), 15
- **E.F. JUNIPER et al.** Development and validation of a questionnaire to measure asthma control. *Eur Respir J.*, 1999, vol. 14 (4), 902-907

- **R.A. NATHAN et al.** Development of the asthma control test: a survey for assessing asthma control. *J Allergy Clin Immunol*, 2004, vol. 113 (1), 59-65
- **M. SCHATZ et al.** Asthma Control Test: reliability, validity, and responsiveness in participants not previously followed by asthma specialists. *J Allergy Clin Immunol*, 2006, vol. 117 (3), 549-56
- **G.S. SHAPIRO et al.** Comparable bronchodilation with hydrofluoroalkane 134a (HFA) albuterol and chlorofluorocarbons 11/12 (CFC) albuterol in children with asthma. *J Asthma*, 2000, vol. 37 (8), 667-75